# EUROPEAN PATENT SPECIFICATION

(11) **EP 3 383 888 B1**
(45) Date of publication and mention of the grant of the patent: **07.06.2023**
(21) Application number: 16805776.8
(22) Date of filing: 01.12.2016
(51) Int. Cl.: C07K 14/32, C12N 9/02, C12N 9/90

(54) **NEW PEPTIDES HAVING ANTIMICROBIAL ACTIVITY AND NEW ENZYME CAPABLE OF CONVERTING L-CONFIGURED RESIDUE IN D-CONFIGURED AMINO ACID IN A PEPTIDE**
NEUE PEPTIDE MIT ANTIMIKROBIELLER AKTIVITÄT UND NEUES ENZYM ZUR UMWANDLUNG VON L-KONFIGURIERTEM RÜCKSTAND IN D-KONFIGURIERTE AMINOSÄURE IN EINEM PEPTID
NOUVEAUX PEPTIDES AYANT UNE ACTIVITÉ ANTIMICROBIENNE ET NOUVELLE ENZYME CAPABLE DE CONVERTIR UN RESTE À CONFIGURATION EN L EN ACIDE AMINÉ À CONFIGURATION EN D DANS UN PEPTIDE

(30) Priority: 02.12.2015 WO PCT/EP2015/306909; 12.08.2016 WO PCT/EP2016/183955
(43) Date of publication of application: 10.10.2018
(73) Proprietor: Institut national de recherche pour l'agriculture, l'alimentation et l'environnement, 75007 Paris (FR)
(72) Inventor: BENJDIA, Alhosna, 94450 Limeil Brevannes (FR); GUILLOT, Alain, 91470 Forges Les Bains (FR); BERTEAU, Olivier, 78350 Jouy en Josas (FR)
(74) Representative: Cabinet Becker et Associés
(86) International application number: PCT/EP2016/079362
(87) International publication number: WO 2017/093366

(56) References cited:
- BUTCHER ET AL.: J BACTERIOL, vol. 189, 2007, page 8616, XP055256513, cited in the application
- Anonymous: "UNIPROT:Q45596", , 14 April 2009 (2009-04-14), XP055256737, Retrieved from the Internet: URL:http://ibis.internal.epo.org/exam/dbfe tch.jsp?id=UNIPROT:Q45596 [retrieved on 2016-03-09]
- FEITELSON ET AL: "Parallel epigenetic and genetic changes in the pathogenesis of hepatitis virus-associated hepatocellular carcinoma", CANCER LETTERS, NEW YORK, NY, US, vol. 239, no. 1, 28 July 2006 (2006-07-28) , pages 10-20, XP005525651, ISSN: 0304-3835, DOI: 10.1016/J.CANLET.2005.07.009
- ALHOSNA BENJDIA ET AL: "Post-translational modification of ribosomally synthesized peptides by a radical SAM epimerase in Bacillus subtilis", NATURE CHEMISTRY, vol. 9, no. 7, 1 July 2017 (2017-07-01), pages 698-707, XP55600299, London ISSN: 1755-4330, DOI: 10.1038/nchem.2714

## Description

### FIELD OF THE INVENTION

The present disclosure relates to the medicine, in particular the antimicrobial activity of peptides and to the enzymology.

### BACKGROUND OF THE INVENTION

Antimicrobial resistance threatens the effective prevention and treatment of an ever-increasing range of infections caused by bacteria, parasites, viruses and fungi. It is an increasingly serious threat to global public health that requires action across all government sectors and society. Antimicrobial resistance is present in all parts of the world. New resistance mechanisms emerge and spread globally.

In consideration of the constant problems due to the antimicrobial resistance, there is a strong need of new classes of antimicrobials. In particular, antimicrobial peptides are of high interest. These peptides are generally potent. In addition, they often offer a better selectivity and specificity than small molecules generally used as therapeutics.

Nevertheless, a major limitation to their use and therapeutic development is associated with their half-life. Indeed, it is commonly decreased by the action of proteases and peptidases which are present in organisms.

Therefore, numerous strategies have been developed in order to reduce the peptide degradation such as C-amidation and N-acetylation against exopeptidase, introduction of non-natural amino acids or of D-configured amino acids. However, these strategies can only be used in the context of synthesized peptides and are not suitable for recombinant production of peptides. In particular, there is no easy way for introducing D-configured amino acid in a peptide, despite the interest of such a modification. Indeed, D-configured amino acid presents the same properties than natural amino acids but are usually more resistant to proteases and even could be less immunogen.

In conclusion, any new class of antimicrobials is of great interest, especially antimicrobial peptides. In addition, any process suitable for modifying L-configured amino acid into D-configured amino acid in a peptide would be highly valuable in this context.

Butcher et al (2007, J Bacteriol, 189, 8676) identified an operon YydFGHIJ which appeared to induce LiaRS expression. Uniprot : Q45596 discloses the amino acid sequences of a yydF peptide. Feitelson et al (2006, Cancer Letters, 239, 10-20) relates to epigenetic and genetic changes.

### SUMMARY OF THE INVENTION

The scope of the present invention is defined by the claims and any information that does not fall within the claims is provided for information only.

The inventors identified an antimicrobial peptide derived from bacteria, namely from *Bacillus subtilis* which possesses D-amino acid residues. Therefore, the inventors discovered a new class of bacteriocins. This peptide has the specificity to present the antimicrobial effect only when some of its amino acids are in D-configuration. This peptide can be prepared by an enzyme derived from the same bacteria which presents the property of changing the configuration of amino acids in a peptide from an L-configuration to a D-configuration. Therefore, this enzyme can be useful for converting L-configured amino acids into D-configured amino acid in peptides, in particular those prepared by recombinant production.

Accordingly the present disclosure relates to an antimicrobial peptide having at least two D-configured amino acids, comprising a sequence of 17 residues having at least 40 % of identity with the sequence in positions 33 to 49 of SEQ ID No 1 and said sequence comprising the sequences W-Y-F-[V/I/A] (SEQ ID No 71) and W-[I/V/A]-X4-G-S (SEQ ID No 69), wherein X4 is any amino acid, and a connecting sequence between SEQ ID No 69 and SEQ ID No 71 of 4-8 amino acids, wherein the residues [V/I/A] of SEQ ID Nos 69 and 71 have a D-configuration.

Preferably, the peptide comprises, essentially consists in or consists in a sequence of W-Y-F-[V/I/A]-[K/R]-Xa-Xb-Xc-N-R-W-[V/I/A]-Xd-G-S-Xe-H (SEQ ID No 72), wherein Xa, Xb and Xc are a polar amino acid, Xd is an aliphatic amino acid and Xe any amino acid. More preferably, the peptide comprises, essentially consists in or consists a sequence of W-Y-F-[V/I/A]-[K/R]-[S/N]-[S/Q/K]-[E/K/S/Q]-N-R-W-[V/I/A]-[L/V/A]-G-S-[A/G]-H (SEQ ID No 73).

In a preferred aspect, the peptide comprises, essentially consists in or consists in a sequence selected from the group consisting of SEQ ID Nos 61-68.

In one aspect, the peptide comprises a sequence of 24 residues having at least 40 % of identity with the sequence in positions 26 to 49 of SEQ ID No 1 and said sequence comprising the sequences N/D-D-L-W-Y-F-[V/I/A] (SEQ ID No 21) and W-[I/V/A]-X4-G-S (SEQ ID No 69), wherein X4 is any amino acid, and a connecting sequence between SEQ ID No 21 and SEQ ID No 69 of 4-7 amino acids.

Preferably, the peptide comprises the sequences L-X1-X2-X3-N-D-L-W-Y-F-V/I (SEQ ID No 23), and W-I/V-X4-G-S (SEQ ID No 22), wherein X1, X2, X3 and X4 are any amino acid. More preferably, X1 is Alanine, Aspartic acid or Glutamic acid, X2 is Lysine, Asparagine or Aspartic acid and X3 is Valine, Isoleucine or Glutamine.

Preferably, the peptide comprises the sequences L-A-K-V-N-D-L-W-Y-F-V (SEQ ID No 24), and W-I/V-X4-G-S (SEQ ID No 22), wherein X4 is a hydrophobic amino acid, preferably Leucine, Valine, Alanine or Methionine.

Preferably, the Valine or Isoleucine in the sequence N/D-D-L-W-Y-F-[**V/I/A]** (SEQ ID No 21) has a D-configuration and the Isoleucine or Valine in the sequence W-**I/V**-X4-G-S (SEQ ID No 22) has a D-configuration.

In a preferred aspect, the peptide comprises, consists essentially of or consists of the sequence of SEQ ID No 20 and the Valine in position 19 is in D-configuration and the isoleucine in position 27 is in D-configuration.

The present disclosure also relates to a pharmaceutical composition comprising a peptide according to the present disclosure.

The present disclosure further relates to a peptide according to the present disclosure for use as a drug, preferable as antimicrobial, more preferably as antibacterial. It relates to the use of a peptide according to the present disclosure for the manufacture of a medicament, preferably an antimicrobial, more preferably an antibacterial. The present disclosure relates to a method for treating a subject in need thereof, comprising administering a therapeutic amount of a peptide according to the present disclosure.

The present disclosure relates to an *in vitro* method for preparing a peptide according to the present disclosure, wherein the method comprises a step of contacting a peptide comprising a sequence of 17 residues having at least 40 % of identity with the sequence in positions 33 to 49 of SEQ ID No 1 and said sequence comprising the sequences W-Y-F-[V/I/A] (SEQ ID No 71) and W-[I/V/A]-X4-G-S (SEQ ID No 69), wherein X4 is any amino acid, and a connecting sequence between SEQ ID No 69 and SEQ ID No 71 of 4-8 amino acids, with a radical SAM peptide epimerase having an amino acid sequence having at least 30 % of identity with SEQ ID No 25 and in which 70% of the positions 14-15, 18, 20-24, 27, 58-60, 63, 83, 84, 87-90, 112, 115, 117, 120, 150, 152, 169, 176, 180, 181, 183, 204, 206-208, 214, 217, 220-225, 228, 230, 252, 254, 262, 289, 292, 296, and 309 of SEQ ID No 25 are conserved.

In one aspect, the peptide comprises a sequence of 24 residues having at least 40 % of identity with the sequence in positions 26 to 49 of SEQ ID No 1 and said sequence comprising the sequences N/D-D-L-W-Y-F-[V/I/A] (SEQ ID No 21) and W-I/V-X4-G-S (SEQ ID No 22), wherein X4 is any amino acid, and a connecting sequence between SEQ ID No 21 and SEQ ID No 22 of 4-7 amino acids.

It further relates to the use of a radical SAM peptide epimerase having an amino acid sequence having at least 30 % of identity with SEQ ID No 25 and in which 70% of the positions 14-15, 18, 20-24, 27, 58-60, 63, 83, 84, 87-90, 112, 115, 117, 120, 150, 152, 169, 176, 180, 181, 183, 204, 206-208, 214, 217, 220-225, 228, 230, 252, 254, 262, 289, 292, 296, and 309 of SEQ ID No 25 are conserved for preparing a peptide according to the present disclosure.

The present disclosure also relates to a recombinant host cell comprising a heterologous nucleic acid encoding a peptide comprising a sequence of 17 residues having at least 40 % of identity with the sequence in positions 33 to 49 of SEQ ID No 1 and said sequence comprising the sequences W-Y-F-[V/I/A] (SEQ ID No 71) and W-[I/V/A]-X4-G-S (SEQ ID No 69), wherein X4 is any amino acid, and a connecting sequence between SEQ ID No 69 and SEQ ID No 71 of 4-8 amino acids, and a heterologous nucleic acid encoding a epimerase having an amino acid sequence having at least 30 % of identity with SEQ ID No 25 and in which 70% of the positions 14-15, 18, 20-24, 27, 58-60, 63, 83, 84, 87-90, 112, 115, 117, 120, 150, 152, 169, 176, 180, 181, 183, 204, 206-208, 214, 217, 220-225, 228, 230, 252, 254, 262, 289, 292, 296, and 309 of SEQ ID No 25 and being able to co-express the peptide and the epimerase. In one aspect, the peptide comprises a sequence of 24 residues having at least 40 % of identity with the sequence in positions 26 to 49 of SEQ ID No 1 and said sequence comprising the sequences N/D-D-L-W-Y-F-[V/I/A] (SEQ ID No 21) and W-I/V-X4-G-S (SEQ ID No 22), wherein X4 is any amino acid, and a connecting sequence between SEQ ID No 21 and SEQ ID No 22 of 4-7 amino acids

Finally, the present disclosure relates to a method for preparing a peptide according to the present disclosure, wherein the method comprises culturing a recombinant host cell according to the present disclosure in conditions suitable for the co-expression the peptide and the epimerase and recovering the peptide having at least one D-configured amino acid or synthetic methods used for peptide synthesis, which allow the assembly of L- and D-configured amino acids.

### DETAILED DESCRIPTION OF THE INVENTION

By studying the yydFGHIJ operon of *Bacillus subtilis,* the inventors identified firstly a new class of antimicrobial peptides and, secondly, an epimerase capable of converting *in vitro* L-configured amino acid into D-configured residue in the peptide. Therefore, this epimerase is useful for peptides engineering.

### Antimicrobial peptide

In a search for genes involved in the regulation of the two-component system LiaRS presumably involved in sensing bacterial cell-wall integrity, Butcher et al (2007, J Bacteriol, 189, 8616) identified an operon YydFGHIJ which appeared to induce LiaRS expression. However, no component of this operon could be isolated or investigated *in vitro.*

The inventors showed that the operon produces a peptide YydF and that this peptide is post-translationaly modified by the YydG enzyme which, very surprisingly, encodes a novel class of radical SAM epimerase. In addition, the inventors discovered that the YydF peptide has antimicrobial activity but only if it comprises D-configured amino acids. In absence of D-configured amino acids, the peptide is devoid of this antimicrobial activity, a feature unknow to date in bioactive peptides. More particularly, two D-configured amino acids are required for this antimicrobial activity.

Accordingly, in one aspect, the present disclosure relates to a peptide having at least one D-configured amino acid, preferably two D-configured amino acids, comprising a sequence of 17 residues having at least 40 % of identity with the sequence in positions 33 to 49 of SEQ ID No 1. In particular, the peptide may comprise a sequence of 17 residues having at least 45, 50, 55, 60, 70, 80, 85, 90, 95, 98 or 99 % of identity with the sequence in positions 33 to 49 of SEQ ID No 1. Alternatively, the peptide may comprise a sequence of 17 residues in positions 33 to 49 of SEQ ID No 1, and 1, 2, 3, 4, 5, 6, 7, 8, 9 or 10 substitutions, additions or deletions.

In one preferred aspect, the peptide comprises a sequence in which the residues in positions 33-35, 40-43, 46 and 47 of SEQ ID No 1 are conserved. In an additional aspect, residues in positions 36 and 44 of SEQ ID No 1 are selected from the group consisting of V, I and A, preferably V and I.

In a preferred aspect, the present disclosure relates to a peptide having at least two D-configured amino acids, comprising a sequence of 17 residues having at least 40 % of identity with the sequence in positions 33 to 49 of SEQ ID No 1 and including the sequence W-[I/V/A]-X4-G-S (SEQ ID No 69), wherein X4 is a hydrophobic amino acid, preferably Leucine, Valine or Alanine. In particular, the peptide may comprise a sequence of 17 residues having at least 40, 45, 50, 55, 60, 70, 80, 85, 90 or 95 % of identity with the sequence in positions 33 to 49 of SEQ ID No 1. Optionally, the peptide further comprises the sequence W-Y-F-[V/I/A] (SEQ ID No 71). Preferably, the amino acids [V/I/A] of SEQ ID Nos 69 and 71 are D-configured amino acids. In the peptide sequence, when considering orientation from the N-terminal end to the C-terminal end, the sequence W-Y-F-[V/I/A] (SEQ ID No 71) is before the sequence W-[I/V/A]-X4-G-S (SEQ ID No 69), preferably W-[I/V]-X4-G-S (SEQ ID No 22). In a preferred aspect, the motif W-Y-F-[V/I/A] (SEQ ID No 71) is separated from the motif W-[I/V/A]-X4-G-S (SEQ ID No 69), preferably W-[I/V]-X4-G-S (SEQ ID No 22), by a linking sequence comprising from four to eight amino acids, preferably a linking sequence of five or seven amino acids, preferably of six amino acids. In a most prefered aspect, the peptide comprises a sequence of W-Y-F-[V/I/A]-[K/R]-Xa-Xb-Xc-N-R-W-[V/I/A]-Xd-G-S-Xe-H (SEQ ID No 72), wherein Xa, Xb and Xc are a polar amino acid, Xd is an aliphatic amino acid and Xe is any amino acid. Preferably, Xa is selected from the group consisting of S, N, C and T, more preferably is S or N. Preferably, Xb is selected from the group consisting of S, N, T, Q, D, E, K, R and H, more preferably selected from the group consisting of S, N, Q and K, still more preferably S, Q and K. Preferably, Xc is selected from the group consisting of S, N, T, Q, D, E, K, R and H, more preferably selected from the group consisting of S, N, Q, E, R and K, still more preferably E, K, S and Q. Preferably, Xd is selected from the group consisting of L, I, V A, more preferably L, V and A. Preferably, Xe is selected from the group consisting of A, G or S, more preferably A or G.

In a very specific aspect, the peptide essentially consists in or consists in a sequence of W-Y-F-[V/I/A]-[K/R]-[S/N]-[S/Q/K]-[E/K/S/Q]-N-R-W-[V/I/A]-[L/V/A]-G-S-[A/G]-H (SEQ ID No 73).

In an aspect of the disclosure, the peptide comprises, essentially consists in or consists in a sequence selected from the group consisting of SEQ ID Nos 61-68.

In one aspect, the present disclosure relates to a peptide having at least one D-configured amino acid, preferably two D-configured amino acids, comprising a sequence of 24 residues having at least 40 % of identity with the sequence in positions 26 to 49 of SEQ ID No 1. In particular, the peptide may comprise a sequence of 24 residues having at least 45, 50, 60, 70, 80, 85, 90, 95, 98 or 99 % of identity with the sequence in positions 26 to 49 of SEQ ID No 1. Alternatively, the peptide may comprise a sequence of 24 residues in positions 26 to 49 of SEQ ID No 1, and 1, 2, 3, 4, 5, 6, 7, 8, 9 or 10 substitutions, additions or deletions.

In a preferred aspect, the peptide comprises a sequence in which the residues in positions 26, 31-35, 43, 46 and 47 of SEQ ID No 1 are conserved. In an additional aspect, one or several residues in positions 23, 25, 27-30, 36, 37, 38, 41, 42, 44, 48 and 49 of SEQ ID No 1 are also conserved. For instance, 1-11 residues in positions 23, 25, 27-30, 36, 37, 38, 41, 42, 44, 48 and 49 of SEQ ID No 1 can be conserved, especially 1, 2, 3, 4, 5, 6, 7, 8, 9, 10 or 11 residues. More preferably, the residues in positions 23, 25, 27-30, 36, 37, 38, 41, 42, 44, 48 and 49 of SEQ ID No 1 are conserved. Still more preferably, the residues in positions 25, 29, and 38 of SEQ ID No 1 are conserved.

Then, a peptide according to the present disclosure comprises the sequences N/D-D-L-W-Y-F-[V/I/A] (SEQ ID No 21), in particular L-X1-X2-X3-N-D-L-W-Y-F-V/I (SEQ ID No 23), or/and W-I/V/AX4-G-S (SEQ ID No 22), wherein X1, X2, X3 and X4 are any amino acid. Optionally, the peptide comprises both sequences. Optionally, the sequence W-I/V/A-X4-G-S (SEQ ID No 69) is the sequence W-I/V-X4-G-S (SEQ ID No 22). Preferably, X1 is Alanine, Aspartic acid or Glutamic acid, preferably Alanine and Aspartic acid, more preferably Alanine. Preferably, X2 is Lysine, Asparagine or Aspartic acid, preferably Lysine or Asparagine, more preferably Lysine. Preferably, X3 is Valine, Isoleucine or Glutamine, preferably Valine or Isoleucine, more preferably Valine. Preferably, X4 is a hydrophobic amino acid, preferably Leucine, Valine, Alanine or Methionine. In a particular aspect, X1 is Alanine and Aspartic acid, preferable Alanine, X2 is Lysine or Asparagine, preferably Lysine, X3 is Valine or Isoleucine, preferably Valine, and X4 is a hydrophobic amino acid, preferably Leucine, Valine or Alanine. In a preferred aspect, the peptide comprises the sequences L-A-K-V-N-D-L-W-Y-F-V (SEQ ID No 24), and W-I/V-X4-G-S (SEQ ID No 22), wherein X4 is a hydrophobic amino acid, preferably Leucine, Valine or Alanine. "I/V" or "V/I" means herein Isoleucine or Valine. "N/D "means herein Asparagine or Aspartic Acid. "I/V/A" means herein Isoleucine, Valine or Alanine.

In a preferred aspect, in the sequence W-I/V-X4-G-S (SEQ ID No 22), I/V is an Isoleucine (SEQ ID No 57). Alternatively, in the sequence W-I/V-X4-G-S (SEQ ID No 22), I/V is a Valine (SEQ ID No 58). In another aspect, W-I/V/A-X4-G-S (SEQ ID No 69) is W-A-X4-G-S (SEQ ID No 70). Preferably, X4 is a hydrophobic amino acid, more preferably Leucine, Valine, Alanine or Methionine, still more preferably Leucine, Valine, or Alanine.

In a preferred aspect, in the sequence N/D-D-L-W-Y-F-[V/I/A] (SEQ ID No 21), and N/D is an Asparagine and I/V is a Valine. Alternatively, in the sequence N/D-D-L-W-Y-F-[V/I/A] (SEQ ID No 21), and N/D is an Aspartic Acid and I/V is an Isoleucine.

In the peptide sequence, when considering orientation from the N-terminal end to the C-terminal end, the sequence N/D-D-L-W-Y-F-[V/I/A] (SEQ ID No 21), in particular L-X1-X2-X3-N-D-L-W-Y-F-V/I (SEQ ID No 23), is before the sequence W-I/V-X4-G-S (SEQ ID No 22).

In a preferred aspect, the motif N/D-D-L-W-Y-F-[V/I/A] (SEQ ID No 21) is separated from the motif W-I/V-X4-G-S (SEQ ID No 22) by a linking sequence comprising from four to seven amino acids, preferably a linking sequence of five or six amino acids, preferably of five amino acids. In a most prefered aspect, the peptide comprises a sequence of N/D-D-L-W-Y-F-V/I-(X)₅-W-I/V-X4-G-S (SEQ ID No 59) or N/D-D-L-W-Y-F-V/I-(X)₆-W-I/V-X4-G-S (SEQ ID No 60), X4 having the same meaning than above.

Then, in a preferred aspect, the present disclosure relates to a peptide having at least one D-configured amino acid, comprising a sequence of 24 residues having at least 40 % of identity with the sequence in positions 26 to 49 of SEQ ID No 1 and including the sequences L-A-K-V-N-D-L-W-Y-F-V (SEQ ID No 24), and/or W-I/V-X4-G-S (SEQ ID No 22), wherein X4 is a hydrophobic amino acid, preferably Leucine, Valine or Alanine. In particular, the peptide may comprise a sequence of 24 residues having at least 45, 50, 60, 70, 80, 85, 90 or 95 % of identity with the sequence in positions 26 to 49 of SEQ ID No 1. In a preferred aspect, one or several residues in positions 23, 25, 27-30, 36, 37, 38, 41, 42, 44, 48 and 49 of SEQ ID No 1 are also conserved, especially 1, 2, 3, 4, 5, 6, 7, 8, 9, 10 or 11 residues.

The peptide comprises at least one D-configured amino acid, preferably two D-configured amino acids. For instance, it may comprise 1, 2, 3, 4 or 5 D-configured amino acids. Optionally, the D-configured amino acids can be any amino acid, preferably an amino acid selected from the group consisting of I, V, A, N, S and T, more preferably selected from the group consisting of I, V and A, still more preferably selected from the group consisting of I and V.

In a particular aspect, the Valine, Isoleucine or Alanine in the sequence N/D-D-L-W-Y-F-F-[**V/I/A]** (SEQ ID No 21) has a D-configuration and/or the Isoleucine, Valine or Alanine in the sequence W-[I/V/A]-X4-G-S (SEQ ID No 69), preferably W-**I/V**-X4-G-S (SEQ ID No 22), has a D-configuration. In a preferred aspect, the Valine, Isoleucine or Alanine in the sequence N/D-D-L-W-Y-F-[**V/I/A]** (SEQ ID No 21) and the Isoleucine, Valine or Alanine in the sequence W-[I/V/A]-X4-G-S (SEQ ID No 69), preferably W-**I/V**-X4-G-S (SEQ ID No 22), have a D-configuration. In a preferred aspect, the Valine or Isoleucine in the sequence of N/D-D-L-W-Y-F-**V/I**-(X)₅-W-**I/V**-X4-G-S (SEQ ID No 59) or N/D-D-L-W-Y-F-**V/I**-(X)₆-W-**I/V**-X4-G-S (SEQ ID No 60) have a D-configuration. Optionally, the peptide may comprise additional D-configured amino acid.

Preferably, the length of the peptide is no more about 65 amino acids, more preferably no more than 50 amino acids, in particular can be from about 15 to about 50 amino acids, for instance from about 17 to about 50 amino acids, from about 18 to about 50 amino acids, from about 17 to about 40, from about 20 to about 40 or from about 25 to about 35 amino acids.

The peptide can comprise, consist essentially of or consist of a sequence selected from the group consisting of the amino acid sequences shown in SEQ ID Nos 1-19 or a funtional fragment thereof comprising the sequences N/D-D-L-W-Y-F-[V/I/A] (SEQ ID No 21), in particular L-X1-X2-X3-N-D-L-W-Y-F-V/I (SEQ ID No 23), and/or W-I/V-X4-G-S (SEQ ID No 22), more preferably L-A-K-V-N-D-L-W-Y-F-V (SEQ ID No 24), and W-I/V-X4-G-S (SEQ ID No 22). By funtional is intended having an antimicrobial activity, especially an antibacterial activity.

In a very particular aspect, the peptide comprises, consists essentially of or consists of the sequence of SEQ ID No 20 and the Valine in position 19 is in D-configuration and/or the isoleucine in position 27 is in D-configuration. Preferably, both the Valine in position 19 and the isoleucine in position 27 are in D-configuration.

In another very particular aspect, the peptide has the sequence of SEQ ID No 1 and the Valine in position 36 is in D-configuration and/or the isoleucine in position 44 is in D-configuration. Preferably, both the Valine in position 36 and the isoleucine in position 44 are in D-configuration.

In a particular aspect, the peptide is not found in nature. The peptide is a non-natural peptide.

The N- and C-termini of the peptides described herein may be optionally protected against proteolysis. In a preferred aspect, the N-terminus may be in the form of an acetyl group, and/or the C-terminus may be in the form of an amide group. In a preferred aspect, the peptide has a free C-terminal end.

Alternatively or in addition, internal modifications of the peptides to be resistant to proteolysis are also envisioned, e.g. wherein at least a -CONH- peptide bond is modified and replaced by a (CH2NH) reduced bond, a (NHCO) retro-inverso bond, a (CH2-O) methylene-oxy bond, a (CH2-S) thiomethylene bond, a (CH2CH2) carba bond, a (CO-CH2) cetomethylene bond, a (CHOH-CH2) hydroxyethylene bond), a (N-N) bound, a E-alcene bond or also a -CH=CH-bond.

For instance, the peptide may be modified by acetylation, acylation, amidation, cross-linking, cyclization, disulfide bond formation, formation of covalent cross-links, formation of cysteine, formation of pyroglutamate, formylation, gamma-carboxylation, glycosylation, GPI anchor formation, hydroxylation, iodination, methylation, myristylation, oxidation, phosphorylation, and the like.

The peptide according to the disclosure may comprise one or more amino acids which are rare amino acids in particular hydroxyproline, hydroxylysine, allohydroxylysine, 6-N-methylysine, N-ethylglycine, N-methylglycine, N-ethylasparagine, allo-isoleucine, N-methylisoleucine, N-methylvaline, pyroglutamine, aminobutyric acid; or synthetic amino acids in particular ornithine, norleucine, norvaline and cyclohexyl-alanine.

Optionally, the peptide can be linked to additional moiety, optionally through a linker or spacer (e.g., diglycine). Optionally, the peptide can be part of a protein fusion. The additional moiety can be a moiety facilitating its cellular uptake or entry, in particular a PTD (protein transduction domain) or Cell Penetrating Peptide; a homing peptide; a stabilizing agent such as PEG (polyethyleneglycol), oligo-N-methoxy-ethylglycine (NMEG), albumin, an albumin-binding protein or an immunoglobulin Fc domain; an affinity tag such as an immune-tag, biotin, lectin, or chelator; a purification tag such as a His-tag; a detectable label such as an optical tag, a chelated lanthamide, a fluorescent dye, or a FRET acceptor/donor; a targeting moiety; a secretion signal peptide; or a combination thereof.

The additional moiety can be added either at the N-terminal end or C-terminal end of the peptide. Preferably, the additional moiety is added either at the N-terminal end of the peptide.

In another aspect of the disclosure, peptides are covalently bound to a polyethylene glycol (PEG) molecule by their C-terminal terminus or a lysine residue, notably a PEG of 1500 or 4000 MW, for a decrease in urinary clearance and in therapeutic doses used and for an increase of the half-life in blood plasma. In yet another aspect, peptide half-life is increased by including the peptide in a biodegradable and biocompatible polymer material for drug delivery system forming microspheres. Polymers and copolymers are, for instance, poly(D,L-lactide-co-glycolide) (PLGA) (as illustrated in US2007/0184015, SoonKap Hahn et al).

The disclosure also encompasses the pharmaceutically acceptable salts of a peptide according to the disclosure. Pharmaceutically acceptable salts may, for example, be salts of pharmaceutically acceptable mineral acids such as hydrochloric acid, hydrobromic acid, sulphuric acid and phosphoric acid; salts of pharmaceutically acceptable organic acids such as acetic acid, citric acid, maleic acid, malic acid, succinic acid, ascorbic acid and tartaric acid; salts of pharmaceutically acceptable mineral bases such as salts of sodium, potassium, calcium, magnesium or ammonium; or salts of organic bases which contain a salifiable nitrogen, commonly used in pharmaceutical technique. The methods for preparing said salts are well known to one of skill in the art.

In a preferred aspect, the peptide is isolated.

### Use of the Antimicrobial peptide

The present disclosure relates to a pharmaceutical composition comprising a peptide as defined above. The pharmaceutical composition may further comprise a pharmaceutically acceptable carrier and/or excipient. By pharmaceutical use is also contemplated the veterinary use. Optionally, the pharmaceutical composition may further comprise another active ingredient, preferably another antimicrobial, more preferably another antibacterial or an anti-inflammatory agent.

The pharmaceutical composition comprising the molecule is formulated in accordance with standard pharmaceutical practice (Lippincott Williams & Wilkins, 2000 and Encyclopedia of Pharmaceutical Technology, eds. J. Swarbrick and J. C. Boylan, 1988-1999, Marcel Dekker, New York) known by a person skilled in the art.

The present disclosure also relates to a peptide as defined above for use as a drug, in particular as an antimicrobial, more preferably as an antibacterial. It further relates to the use of a peptide as defined above for the manufacture of a medicament for use as an antimicrobial, more preferably as an antibacterial. In addition, it relates to a method for treating or preventing an infection, especially a bacterial infection, comprising administering a therapeutically effective amount of a peptide as defined above, thereby treating or preventing an infection.

In a preferred aspect, the peptide as defined above is such that the Valine, Isoleucine or Alanine in the sequence N/D-D-L-W-Y-F-[**V/I/A]** (SEQ ID No 21) and the Isoleucine, Valine or Alanine in the sequence W-[I/V/A]-X4-G-S (SEQ ID No 69), preferably W-**I/V**-X4-G-S (SEQ ID No 22), have a D-configuration. In a very specific aspect, the peptide comprises, consists essentially of or consists of the sequence of SEQ ID No 20 and the Valine in position 19 and the isoleucine in position 27 are in D-configuration. In another very particular aspect, the peptide has the sequence of SEQ ID No 1 and the Valine in position 36 and the isoleucine in position 44 are in D-configuration. In another very particular aspect, the peptide comprises, essentially consists in or consists in a sequence selected from the group consisting of SEQ ID Nos 61-68, wherein the residues in positions 4 and 12 are in D-configuration.

Optionally, the peptide as defined above can be used in combination with another drug, preferably another antimicrobial, more preferably another antibacterial, or an anti-inflammatory agent.

By "treat" or "treatment" is intended that the disease is cured, alleviated or delayed. It includes the preventive or curative treatment.

The term "therapeutically effective amount" as used in the present application is intended an amount of therapeutic agent, administered to a patient that is sufficient to constitute a treatment of infection.

The form of the pharmaceutical compositions, the route of administration, the dosage and the regimen naturally depend upon the condition to be treated, the severity of the illness, the age, weight, and sex of the patient, etc.

The pharmaceutical compositions of the disclosure can be formulated for a topical, oral, parenteral, intranasal, intravenous, intramuscular, subcutaneous or intraocular administration and the like.

For oral administration, the composition can be formulated into conventional oral dosage forms such as tablets, capsules, powders, granules and liquid preparations such as syrups, elixirs, and concentrated drops. Non toxic solid carriers or diluents may be used which include, for example, pharmaceutical grades of mannitol, lactose, starch, magnesium stearate, sodium saccharine, talcum, cellulose, glucose, sucrose, magnesium, carbonate, and the like. For compressed tablets, binders, which are agents which impart cohesive qualities to powdered materials, are also necessary. For example, starch, gelatine, sugars such as lactose or dextrose, and natural or synthetic gums can be used as binders. Disintegrants are also necessary in the tablets to facilitate break-up of the tablet. Disintegrants include starches, clays, celluloses, algins, gums and crosslinked polymers. Moreover, lubricants and glidants are also included in the tablets to prevent adhesion to the tablet material to surfaces in the manufacturing process and to improve the flow characteristics of the powder material during manufacture. Colloidal silicon dioxide is most commonly used as a glidant and compounds such as talc or stearic acids are most commonly used as lubricants.

For transdermal administration, the composition can be formulated into ointment, cream or gel form and appropriate penetrants or detergents could be used to facilitate permeation, such as dimethyl sulfoxide, dimethyl acetamide and dimethylformamide.

For transmucosal administration, nasal sprays, rectal or vaginal suppositories can be used. The active compound can be incorporated into any of the known suppository bases by methods known in the art. Examples of such bases include cocoa butter, polyethylene glycols (carbowaxes), polyethylene sorbitan monostearate, and mixtures of these with other compatible materials to modify the melting point or dissolution rate.

Pharmaceutical compositions according to the disclosure may be formulated to release the active drug substantially immediately upon administration or at any predetermined time or time period after administration.

In a particular aspect, the pharmaceutical composition according to the disclosure comprises 0.001 mg to 10 g of the molecule of the disclosure. Preferably, pharmaceutical composition according to the disclosure comprises 0.01 mg to 1 g of the molecule of the disclosure.

In still another aspect, the present disclosure relates to the use of a peptide according to the disclosure as disinfectant, preservative or pesticide. The term "disinfectant" refers to an antimicrobial activity of the peptide on a surface (for example, walls, doors, medical equipment), a liquid (for example, water) or a gas (for example, an anaesthetic gas). According to one aspect, the peptide according to the disclosure is used for elimination of bacterial biofilms. According to a preferred aspect, the peptide according to the disclosure is used in particular for disinfecting surgical or prosthetic equipment.

In another aspect, the present disclosure relates to a medical device or implant comprising a body having at least one surface coated with or including a peptide according to the disclosure. The present disclosure also relates to a method for preparing a medical device or implant comprising applying a coating of peptide according to the disclosure, or placing in contact, with at least one surface of the device or implant.

This type of medical device or implant and the uses and methods of preparation thereof are described for example in patent application WO 2005/006938.

The surface coated with or including a peptide according to the disclosure may be composed of thermoplastic or polymeric materials such as polyethylene, Dacron, nylon, polyesters, polytetrafluoroethylene, polyurethane, latex, silicone elastomers and the like, or of metallic materials such as gold. In a particular aspect, the peptide of the disclosure is covalently attached to a functionalized surface, preferably a metallic surface, via its N-terminal or C-terminal end. Optionally, the peptide may be attached to the surface through a spacer arm.

Preferably, the surface may be coated with a peptide at a density of 0.4 to 300 mg/cm².

Alternatively, the device or implant, in particular bone and joint prosthetic device, may be coated with a cement mixture comprising a peptide according to the disclosure.

The peptide may be combined with another active molecule, preferably an antibiotic.

The device or implant may be, for example, intravascular, peritoneal, pleural and urological catheters; heart valves; cardiac pacemakers; vascular shunts; coronary stunts; dental implants or orthopaedic or intraocular prosthesis.

### Preparation of the peptide

Peptides described herein can be synthesized using standard synthetic methods known to those skilled in the art, for example chemical synthesis or genetic recombination. In particular, the peptides can be synthesized according to the method originally described by Merrifield.

Examples of chemical synthesis technologies are solid phase synthesis and liquid phase synthesis. As a solid phase synthesis, for example, the amino acid corresponding to the C-terminus of the peptide to be synthesized is bound to a support which is insoluble in organic solvents, and by alternate repetition of reactions, one wherein amino acids with their amino groups and side chain functional groups protected with appropriate protective groups are condensed one by one in order from the C-terminus to the N- terminus, and one where the amino acids bound to the resin or the protective group of the amino groups of the peptides are released, the peptide chain is thus extended in this manner. Solid phase synthesis methods are largely classified by the tBoc method and the Fmoc method, depending on the type of protective group used. Typically used protective groups include tBoc (t-butoxycarbonyl), CI-Z (2-chlorobenzyloxycarbonyl), Br-Z (2-bromobenzyloyycarbonyl), Bzl (benzyl), Fmoc (9-fluorenylmcthoxycarbonyl), Mbh (4, 4'-dimethoxydibenzhydryl), Mtr (4-methoxy-2, 3, 6-trimethylbenzenesulphonyl), Trt (trityl), Tos (tosyl), Z (benzyloxycarbonyl) and Clz-Bzl (2, 6-dichlorobenzyl) for the amino groups; NO2 (nitro) and Pmc (2,2, 5,7, 8-pentamethylchromane-6-sulphonyl) for the guanidino groups); and tBu (t-butyl) for the hydroxyl groups). After synthesis of the desired peptide, it is subjected to the deprotection reaction and cut out from the solid support. Such peptide cutting reaction may be carried with hydrogen fluoride or tri-fluoromethane sulfonic acid for the Boc method, and with TFA for the Fmoc method.

Alternatively, the peptide may be synthesized using recombinant techniques. In this case, a nucleic acid and/or a genetic construct comprising a nucleotide sequence encoding a peptide according to the disclosure is used. Therefore, the present disclosure relates to a nucleic acid and/or a genetic construct comprising a nucleotide sequence encoding a peptide according to the disclosure. The genetic construct comprises a polynucleotide encoding a peptide according to the disclosure as defined herein, and regulatory sequences (such as a suitable promoter(s), enhancer(s), terminator(s), etc.) allowing the expression (*e*.*g*. transcription and translation) of a peptide according to the disclosure in a host cell. Thus, in another aspect, the disclosure relates to a host or host cell that expresses (or that under suitable circumstances is capable of expressing) a peptide of the disclosure; and/or that contains a polynucleotide of the disclosure or genetic construct of the disclosure.

In order to obtain the D-amino acid containing peptide, the peptide can be co-expressed with the peptide epimerase described in this disclosure.

The method of producing the peptide may optionally comprise the steps of purifying said peptide, chemically modifying said peptide, and/or formulating said peptide into a pharmaceutical composition. For instance, the peptide described herein can be prepared by recombinant techniques as a protein fusion with a secretion signal peptide or a purification tag. This secretion signal peptide or purification tag can been cleaved or removed at a further stage of production.

In a particular object of the present disclosure, the method for preparing the peptide comprises providing or synthetizing a peptide as described above with L-configured amino acids and contacting the peptide with an enzyme (i.e., an epimerase), thereby converting at least one L-configured amino acid into D-configured amino acid of said peptide, the epimerase being as defined below.

### Radical SAM epimerase

The inventors identified a radical SAM epimerase called YydG in *Bacillus subtilis* which is able to modify the configuration of amino acids contained in a peptide from a L-configuration to a D-configuration. This enzyme is capable of carrying out the conversion, even in *in vitro* conditions. Its sequence is shown in SEQ ID No 18. In addition, this enzyme is unrelated to other know epimerases, even within the radical SAM enzyme superfamily.

Therefore, the present disclosure relates to an epimerase capable of modifying the configuration of amino acids contained in a peptide from a L-configuration to a D-configuration and comprising an amino sequence having at least 30 % of identity with SEQ ID No 25. Preferably, the epimerase comprises an amino sequence having at least 35, 40, 50, 60, 70, 80, 85, 90, 95, 97.5 or 99 % identity with SEQ ID No 25.

YydG of *Bacillus subtilis* is described in public databases under the following identification numbers: UniProt Q45595; GenelD 937720; GenBank NP_391897.1 and NC_000964.3.

Based on the teaching of the present disclosure, the one skilled in the art can identify other enzymes from microorganisms having the radical SAM epimerase. The polypeptide may be identified and obtained from other sources including microorganisms isolated from nature (e.g., soil, composts, water, etc.) or DNA samples obtained directly from natural materials (e.g., soil, composts, water, etc.) using the probes. Techniques for isolating microorganisms and DNA directly from natural habitats are well known in the art. A polynucleotide encoding the polypeptide may then be obtained by similarly screening a genomic DNA or cDNA library of another microorganism or mixed DNA sample. Once a polynucleotide encoding a polypeptide has been detected, the polynucleotide can be isolated or cloned by utilizing techniques that are known to those of ordinary skill in the art (see, e.g., Sambrook et al., 1989). The one skilled in the art could also use the sequences data already available in databasis. In addition, the person skilled in the art can prepare variants of the epimerase having the amino acid sequence of SEQ ID No 25 by currently used methods. In particular, variants with advantageous properties such as an increased stability (e.g., thermostability), increased production of D-configured amino acid, modifying the specificity and/or the selectivity of the epimerase, and the like.

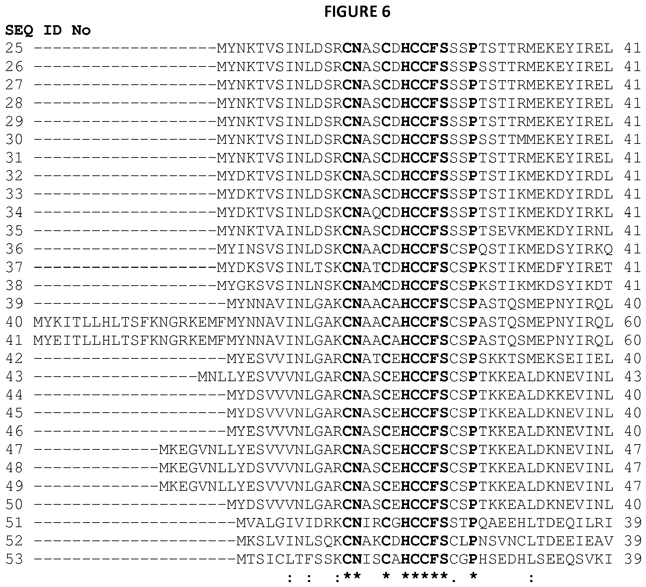

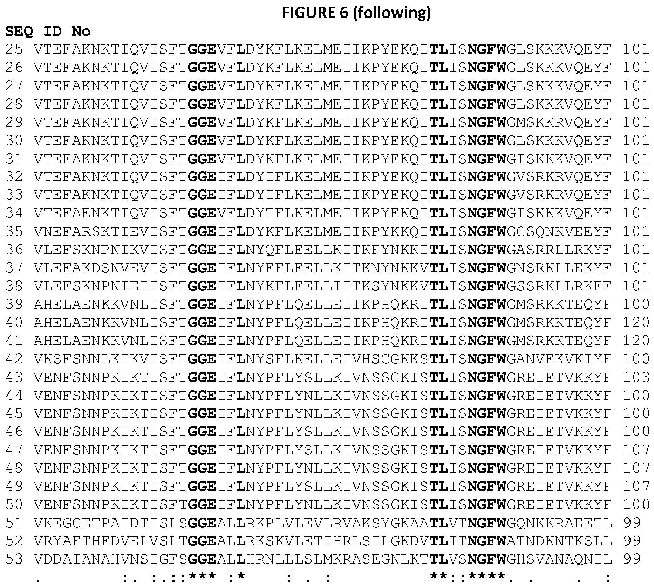

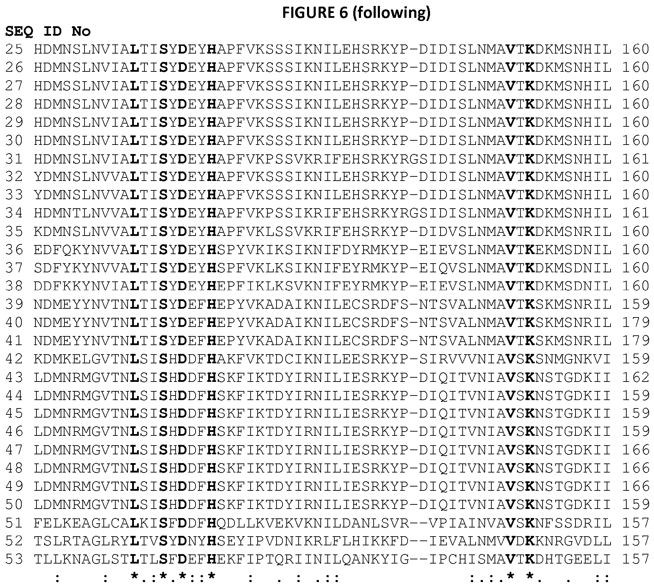

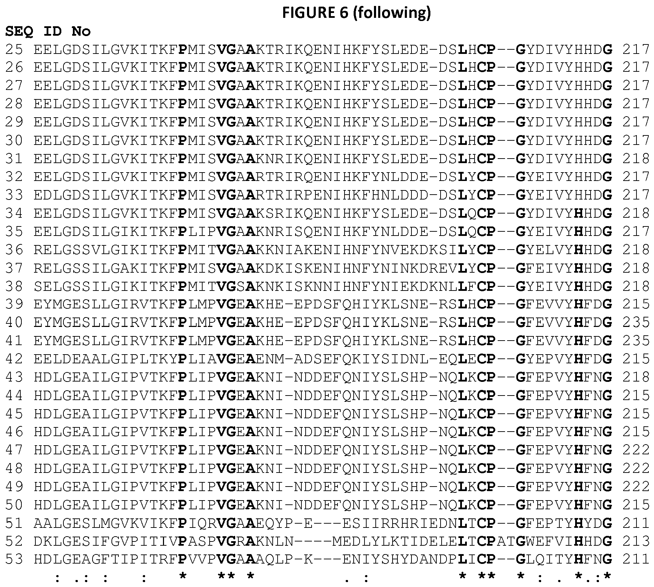

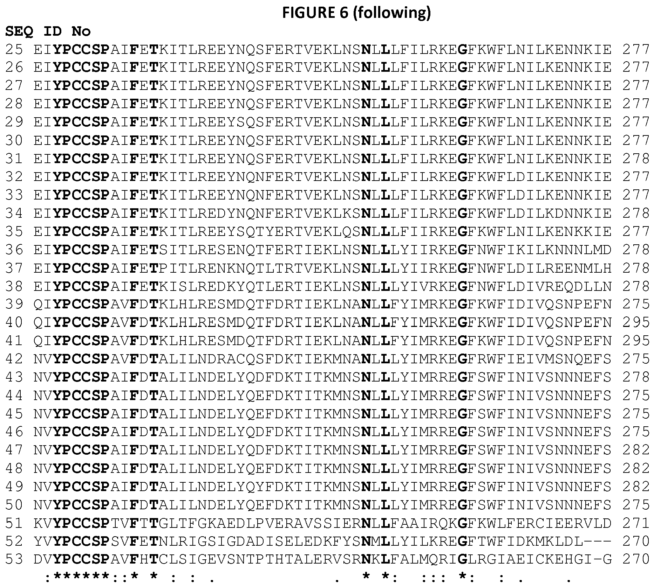

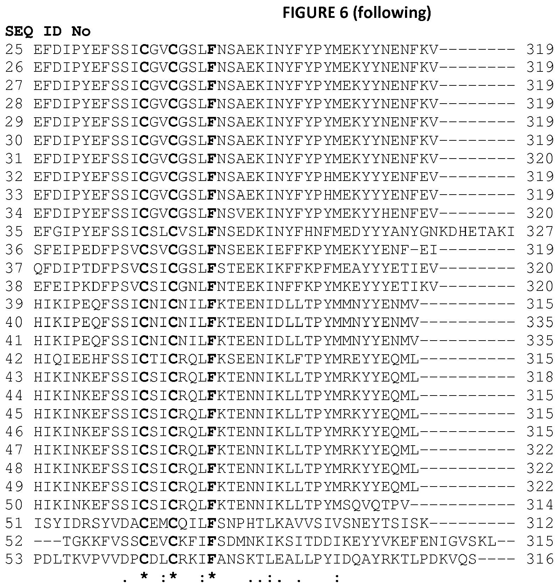

100 % identical sequences in comparison to SEQ ID No 25 are disclosed in Uniprot AOAOC2UHS5, AOAOA1MJP5 and L8AWU7. Other *Bacillus subtilis* strains present sequences with high identity (up to 90 % of identity) and are disclosed in SEQ ID Nos 26-29 and 32. Other *Bacillus* strains present sequences with high identity and are disclosed in SEQ ID Nos 30 and 33-35. Sequences with a significant identity have been identified in *Salinibacillus aidiingensis, Staphylococcus equorum, Staphylococcus pseudintermedius, Staphylococcus epidermidis, Paenibacillus sp, Enterococcus caccae, Enterococcus faecalis, Corynebacterium diphtheria, Streptococcus agalactiae,* and *Bifidobacterium bohemicum* and are disclosed in SEQ ID Nos 31, and 36-52. Figure 6 shows an alignment of the enzymes' sequences.

10 20 30 40 50
MYNKTVSINL DSRCNASCDH CCFSSSPTST TRMEKEYIRE LVTEFAKNKT
60 70 80 90 100
IQVISFTGGE VFLDYKFLKE LMEIIKPYEK QITLISNGFW GLSKKKVQEY
110 120 130 140 150
FHDMNSLNVI ALTISYDEYH APFVKSSSIK NILEHSRKYP DIDISLNMAV
160 170 180 190 200
TKDKMSNHIL EELGDSILGV KITKFPMISV GAAKTRIKQE NIHKFYSLED
210 220 230 240 250
EDSLHCPGYD IVYHHDGEIY PCCSPAIFET KITLREEYNQ SFERTVEKLN
260 270 280 290 300
SNLLLFILRK EGFKWFLNIL KENNKIEEFD IPYEFSSICG VCGSLFNSAE
310
KINYFYPYME KYYNENFKV (SEQ ID No 25)

Positions in bold and underlined are perfectly conserved among the list of sequences disclosed above. Positions in bold and italic are conserved between groups of strongly similar properties among the list of sequences disclosed above. Accordingly, it can be observed that even if the identity percentage is around 30 %, there are a high number of amino acids that are perfectly conserved and well-conserved.

Accordingly, in a preferred aspect, the epimerase has an amino acid sequence having at least 30 % of identity with SEQ ID No 25 and in which 70, 80 or 90 % of the positions in bold and underlined are conserved when the sequence is aligned with SEQ ID No 25 (positions 14-15, 18, 20-24, 27, 58-60, 63, 83, 84, 87-90, 112, 115, 117, 120, 150, 152, 169, 176, 180, 181, 183, 204, 206-208, 214, 217, 220-225, 228, 230, 252, 254, 262, 289, 292, 296, and 309 of SEQ ID No 25). Preferably, 95 % of the positions in bold and underlined are conserved. In a particular aspect, all the positions in bold and underlined are conserved. More preferably, 90 or 95 % of the positions 14-15, 18, 20-24, 27, 58-60, 63, 83, 84, 87-90, 112, 115, 117, 120, 204, 206-208, 214, 217, 220-225, 228, and 230 of SEQ ID No 25 are conserved in the epimerase sequence. In a particular aspect, all the positions 14-15, 18, 20-24, 27, 58-60, 63, 83, 84, 87-90, 112, 115, 117, 120, 204, 206-208, 214, 217, 220-225, 228, and 230 of SEQ ID No 25 are conserved in the epimerase sequence.

In a preferred aspect, the epimerase has an amino acid sequence having at least 30 % of identity with SEQ ID No 25 and one or several segments selected from the segments 13-27, 56-63, 83-90, 112-120, and 204-230 have at least 50 % of identity with the sequence of SEQ ID No 25. In this aspect, the epimerase has in addition, 90 or 95 % of the positions 14-15, 18, 20-24, 27, 58-60, 63, 83, 84, 87-90, 112, 115, 117, 120, 204, 206-208, 214, 217, 220-225, 228, and 230 of SEQ ID No 25 are conserved in the epimerase sequence.

In a particular aspect, the cysteine residues in positions 14, 18, 21, 222 and 223 of SEQ ID No 25 are conserved in the epimerase sequence.

In an aspect, the epimerase has an amino acid sequence comprising, consisting essentially in, or consisting in a sequence having at least 80, 85, 90 or 95 % of identity with a sequence selected among SEQ ID Nos 25-52, preferably among SEQ ID Nos 25-30, 32 and 34, more preferably SEQ ID No 25. The epimerase may have an amino acid sequence comprising, consisting essentially in, or consisting in a sequence selected among SEQ ID Nos 25-52, preferably among SEQ ID Nos 25-30, 32 and 34, more preferably SEQ ID No 25; and having 1, 2, 3, 4, 5, 6, 7, 8, 9 or 10 substitutions, additions or deletions.

A method for testing the capacity of an epimerase to modify the configuration of amino acids contained in a peptide from a L-configuration to a D-configuration is for instance disclosed in details in the example section. For instance, the epimerase is contacted with a peptide having a sequence selected from the group consisting of SEQ ID Nos 1-20 in presence of the co-factor S-adenosyl-L-methionine (SAM) and the production of peptides including a D-configured amino acid is detected. More specifically, the epimerase is contacted with the peptide YydF18-49 of SEQ ID No 20 in presence of the co-factor S-adenosyl-L-methionine (SAM) and the production of peptides including a D-configured amino acid in position 19 and/or 27 is detected.

It is also provided a hybrid polypeptide or fusion polypeptide in which the amino acid sequence of the enzyme as defined above is fused at the N-terminus or the C-terminus of a region of another polypeptide. Techniques for producing fusion polypeptides are known in the art, and include ligating the coding sequences encoding the enzyme and the addition region of another polypeptide so that they are in frame and that expression of the fusion polypeptide is under control of the same promoter(s) and terminator. Fusion polypeptides may also be constructed using intein technology in which fusion polypeptides are created post-translationally (Cooper et al., 1993, EMBO J. 12: 2575-2583; Dawson et al., 1994, Science 266: 776-779).

The addition region of the fusion polypeptide can be selected in order to enhance the stability of the enzyme according to the present disclosure, to promote the secretion (such as a N-terminal hydrophobic signal peptide) of the fusion protein from a cell (such as a bacterial cell or a yeast cell), or to assist in the purification of the fusion protein. More particularly, the additional region can be a tag useful for purification or immobilization of the enzyme. Such a tag is well-known by the person skilled in the art, for instance a His tag (Hiss), a FLAG tag, a HA tag (epitope derived from the Influenza protein haemagglutinin), a maltose-binding protein (MPB), a MYC tag (epitope derived from the human proto-oncoprotein MYC), a STREP tag or a GST tag (small glutathione-S-transferase).

A fusion polypeptide can further comprise a cleavage site between the enzyme and the addition region. Upon secretion of the fusion protein, the site is cleaved releasing the two polypeptides. Examples of cleavage sites include, but are not limited to, the sites disclosed in Martin et al., 2003, J. Ind. Microbiol. Biotechnol. 3: 568-576; Svetina et al., 2000, J. Biotechnol. 76: 245-251; Rasmussen-Wilson et al., 1997, Appl. Environ. Microbiol. 63: 3488-3493; Ward et al., 1995, Biotechnology 13: 498-503; and Contreras et al., 1991, Biotechnology 9: 378-381; Eaton et al., 1986, Biochemistry 25: 505-512; Collins-Racie et al., 1995, Biotechnology 13: 982-987; Carter et al., 1989, Proteins: Structure, Function, and Genetics 6: 240-248; and Stevens, 2003, Drug Discovery World 4: 35-48.

It is further provided a recombinant nucleic acid construct or vector comprising a nucleic acid sequence encoding the epimerase as defined above. More particularly, the nucleic acid construct or vector is suitable for expressing said epimerase. In addition, it is provided a recombinant host cell comprising a nucleic acid, a recombinant nucleic acid construct or a recombinant vector comprising a nucleic acid sequence encoding the epimerase as defined above.

### Nucleic acid constructs

Indeed, the present disclosure relates to a polynucleotide encoding an epimerase of the present disclosure. The nucleic acid can be DNA (cDNA or gDNA), RNA, or a mixture of the two. It can be in single stranded form or in duplex form or a mixture of the two. It can comprise modified nucleotides, comprising for example a modified bond, a modified purine or pyrimidine base, or a modified sugar. It can be prepared by any method known to one skilled in the art, including chemical synthesis, recombination, and mutagenesis.

The present disclosure also relates to nucleic acid constructs comprising a polynucleotide encoding an epimerase according to the present disclosure operably linked to one or more control sequences that direct the expression of the coding sequence in a suitable host cell under conditions compatible with the control sequences. A polynucleotide may be manipulated in a variety of ways to provide for expression of the epimerase. Manipulation of the polynucleotide prior to its insertion into a vector may be desirable or necessary depending on the expression vector. The techniques for modifying polynucleotides utilizing recombinant DNA methods are well known in the art.

The control sequence may include a promoter that is recognized by a host cell or an *in vitro* expression system for expression of a polynucleotide encoding an epimerase of the present disclosure. The promoter contains transcriptional control sequences that mediate the expression of the epimerase. The promoter may be any polynucleotide that shows transcriptional activity in the host cell including mutant, truncated, and hybrid promoters, and may be obtained from genes encoding extracellular or intracellular polypeptides either homologous or heterologous to the host cell. Optionally, the promoter can be inducible.

Examples of suitable promoters in a bacterial host cell are the promoters obtained from the *Bacillus amyloliquefaciens* alpha-amylase gene (amyQ), *Bacillus licheniformis* alpha-amylase gene (amyL), *Bacillus licheniformis* penicillinase gene (penP), *Bacillus stearothermophilus* maltogenic amylase gene (amyM), *Bacillus subtilis* levansucrase gene (sacB), *Bacillus subtilis* xylA and xylB genes, *Bacillus thuringiensis* crylllA gene (Agaisse and Lereclus, 1994, Molecular Microbiology 13: 97-107), *E. coli* lac operon, *E. coli* trc promoter (Egon et al., 1988, Gene 69: 301 -315), *Streptomyces coelicolor* agarase gene (dagA), and prokaryotic beta-lactamase gene (Villa-Kamaroff et al., 1978, Proc. Natl. Acad. Sci. USA 75: 3727-3731), as well as the tac promoter (DeBoer et al., 1983, Proc. Natl. Acad. Sci. USA 80: 21 -25). Further promoters are described in "Useful proteins from recombinant bacteria" in Gilbert et al., 1980, Scientific American 242: 74-94; and in Sambrook et al., 1989. Examples of tandem promoters are disclosed in WO 99/43835.

Examples of suitable promoters in a filamentous fungal host cell are promoters obtained from the genes for *Aspergillus nidulans* acetamidase, *Aspergillus niger* neutral alpha-amylase, *Aspergillus niger* acid stable alpha-amylase, *Aspergillus niger* or *Aspergillus awamori* glucoamylase (glaA), *Aspergillus oryzae* TAKA amylase, *Aspergillus oryzae* alkaline protease, *Aspergillus oryzae* triose phosphate isomerase, *Fusarium oxysporum* trypsin-like protease (WO96/00787), *Fusarium venenatum* amyloglucosidase (WO 00/56900), *Fusarium venenatum* Daria (WO 00/56900), *Fusarium venenatum* Quinn (WO 00/56900), *Rhizomucor miehei* lipase, *Rhizomucor miehei* aspartic proteinase, *Trichoderma reesei* beta-glucosidase, *Trichoderma reesei* cellobiohydrolase I, *Trichoderma reesei* cellobiohydrolase II, *Trichoderma reesei* endoglucanase I, *Trichoderma reesei* endoglucanase II, *Trichoderma reesei* endoglucanase III, *Trichoderma reesei* endoglucanase IV, *Trichoderma reesei* endoglucanase V, *Trichoderma reesei* xylanase I, *Trichoderma* reesei xylanase II, *Trichoderma reesei* beta-xylosidase, as well as the NA2-tpi promoter (a modified promoter from an *Aspergillus* neutral alpha-amylase gene in which the untranslated leader has been replaced by an untranslated leader from an *Aspergillus* triose phosphate isomerase gene; non-limiting examples include modified promoters from an *Aspergillus niger* neutral alpha-amylase gene in which the untranslated leader has been replaced by an untranslated leader from an *Aspergillus nidulans* or *Aspergillus oryzae* triose phosphate isomerase gene; and mutant, truncated, and hybrid promoters thereof.

In a yeast host, useful promoters are obtained from the genes for *Saccharomyces cerevisiae* enolase (ENO-1), *Saccharomyces cerevisiae* galactokinase (GAL1), *Saccharomyces cerevisiae* alcohol dehydrogenase/glyceraldehyde-3-phosphate dehydrogenase (ADH1, ADH2/GAP), *Saccharomyces cerevisiae* triose phosphate isomerase (TPI), *Saccharomyces cerevisiae* metallothionein (CUP1), and *Saccharomyces cerevisiae* 3-phosphoglycerate kinase. Other useful promoters for yeast host cells are described by Romanos et al., 1992, Yeast 8: 423-488.

The control sequence may also be a transcription terminator, which is recognized by a host cell to terminate transcription. The terminator is operably linked to the 3'-terminus of the polynucleotide encoding the polypeptide. Any terminator that is functional in the host cell may be used in the present disclosure.

Preferred terminators for bacterial host cells are obtained from the genes for *Bacillus clausii* alkaline protease (aprH), *Bacillus licheniformis* alpha-amylase (amyL), and *Escherichia coli* ribosomal RNA (rrnB).

Preferred terminators for filamentous fungal host cells are obtained from the genes for *Aspergillus nidulans* anthranilate synthase, *Aspergillus niger* glucoamylase, *Aspergillus niger* alpha-glucosidase, *Aspergillus oryzae* TAKA amylase, and *Fusarium oxysporum* trypsin-like protease.

Preferred terminators for yeast host cells are obtained from the genes for *Saccharomyces cerevisiae* enolase, *Saccharomyces cerevisiae* cytochrome C (CYC1), and *Saccharomyces cerevisiae* glyceraldehyde-3-phosphate dehydrogenase. Other useful terminators for yeast host cells are described by Romanos et al., 1992, supra.

The control sequence may also be an mRNA stabilizer region downstream of a promoter and upstream of the coding sequence of a gene which increases expression of the gene.

Examples of suitable mRNA stabilizer regions are obtained from a *Bacillus thuringiensis* cryIIIA gene (WO 94/25612) and a *Bacillus subtilis* SP82 gene (Hue et ai, 1995, Journal of Bacteriology 177: 3465-3471).

The control sequence may also be a leader, a non-translated region of an mRNA that is important for translation by the host cell. The leader is operably linked to the 5'-terminus of the polynucleotide encoding the epimerase. Any leader that is functional in the host cell may be used.

Preferred leaders for filamentous fungal host cells are obtained from the genes for *Aspergillus oryzae* TAKA amylase and *Aspergillus nidulans* triose phosphate isomerase.

Suitable leaders for yeast host cells are obtained from the genes for *Saccharomyces cerevisiae* enolase (ENO-1), *Saccharomyces cerevisiae* 3-phosphoglycerate kinase, *Saccharomyces cerevisiae* alpha-factor, and *Saccharomyces cerevisiae* alcohol dehydrogenase/glyceraldehyde-3-phosphate dehydrogenase (ADH2/GAP).

The control sequence may also be a polyadenylation sequence, a sequence operably linked to the 3'-terminus of the polynucleotide encoding the epimerase and, when transcribed, is recognized by the host cell as a signal to add polyadenosine residues to transcribed mRNA. Any polyadenylation sequence that is functional in the host cell may be used.

Preferred polyadenylation sequences for filamentous fungal host cells are obtained from the genes for *Aspergillus nidulans* anthranilate synthase, *Aspergillus niger* glucoamylase, *Aspergillus niger* alpha-glucosidase, *Aspergillus oryzae* TAKA amylase, and *Fusarium oxysporum* trypsin-like protease.

Useful polyadenylation sequences for yeast host cells are described by Guo and Sherman, 1995, Mol. Cellular Biol. 15: 5983-5990.

The control sequence may also be a signal peptide coding region that encodes a signal peptide linked to the N-terminus of the epimerase and directs the epimerase into the cell's secretory pathway. The 5'-end of the coding sequence of the polynucleotide may inherently contain a signal peptide coding sequence naturally linked in translation reading frame with the segment of the coding sequence that encodes the epimerase. Alternatively, the 5'-end of the coding sequence may contain a signal peptide coding sequence that is foreign to the coding sequence. A foreign signal peptide coding sequence may be required where the coding sequence does not naturally contain a signal peptide coding sequence. Alternatively, a foreign signal peptide coding sequence may simply replace the natural signal peptide coding sequence in order to enhance secretion of the polypeptide. However, any signal peptide coding sequence that directs the expressed polypeptide into the secretory pathway of a host cell may be used.

Effective signal peptide coding sequences for bacterial host cells are the signal peptide coding sequences obtained from the genes for *Bacillus* NCIB 1 1837 maltogenic amylase, *Bacillus licheniformis* subtilisin, *Bacillus licheniformis* beta-lactamase, *Bacillus stearothermophilus* alpha-amylase, *Bacillus stearothermophilus* neutral proteases (nprT, nprS, nprM), and *Bacillus subtilis* prsA. Further signal peptides are described by Simonen and Palva, 1993, Microbiological Reviews 57: 109-137.

Effective signal peptide coding sequences for filamentous fungal host cells are the signal peptide coding sequences obtained from the genes for *Aspergillus niger* neutral amylase, *Aspergillus niger* glucoamylase, *Aspergillus oryzae* TAKA amylase, *Humicola insolens* cellulase, *Humicola insolens* endoglucanase V, *Humicola lanuginosa* lipase, and *Rhizomucor miehei* aspartic proteinase.

Useful signal peptides for yeast host cells are obtained from the genes for *Saccharomyces cerevisiae* alpha-factor and *Saccharomyces cerevisiae* invertase. Other useful signal peptide coding sequences are described by Romanos et al., 1992, *supra.*

It may also be desirable to add regulatory sequences that regulate expression of the polypeptide relative to the growth of the host cell. Examples of regulatory systems are those that cause expression of the gene to be turned on or off in response to a chemical or physical stimulus, including the presence of a regulatory compound. Regulatory systems in prokaryotic systems include the lac, tac, and trp operator systems. In yeast, the ADH2 system or GAL1 system may be used. In filamentous fungi, the *Aspergillus niger* glucoamylase promoter, *Aspergillus oryzae* TAKA alpha-amylase promoter, and *Aspergillus oryzae* glucoamylase promoter may be used. Other examples of regulatory sequences are those that allow for gene amplification. In eukaryotic systems, these regulatory sequences include the dihydrofolate reductase gene that is amplified in the presence of methotrexate, and the metallothionein genes that are amplified with heavy metals. In these cases, the polynucleotide encoding the polypeptide would be operably linked with the regulatory sequence.

### Expression vectors

The present disclosure also relates to recombinant expression vectors comprising a nucleic acid construct as disclosed above, or a polynucleotide encoding an epimerase of the present disclosure, a promoter, and transcriptional and translational stop signals. The various nucleotide and control sequences may be joined together to produce a recombinant expression vector that may include one or more convenient restriction sites to allow for insertion or substitution of the polynucleotide encoding the epimerase at such sites. Alternatively, the polynucleotide may be expressed by inserting the polynucleotide or a nucleic acid construct comprising the polynucleotide into an appropriate vector for expression.

In a particular aspect, the expression vector may further comprise a polynucleotide encoding a peptide of the present disclosure as disclosed above, operably linked with the control sequences necessary for its expression. More particularly, the control sequences used for the expression of the epimerase and the peptide of the present disclosure are suitable for co-expression in a host cell. Optionally, the polynucleotide encoding the peptide and the polynucleotide encoding the epimerase can be on the control of a single promoter (i.e., operon) or of two promoters which can be the same or different.

Alternatively, the present disclosure relates to a kit comprising a first expression vector comprising a nucleic acid encoding an epimerase of the present disclosure and a second expression vector comprising a nucleic acid encoding a peptide of the present disclosure. In another alternative, the kit can comprise an expression vector comprising a nucleic acid encoding an epimerase of the present disclosure and a nucleic acid encoding a peptide of the present disclosure.

In creating the expression vector, the coding sequence is located in the vector so that the coding sequence is operably linked with the appropriate control sequences for expression.

The recombinant expression vector may be any vector (e.g., a plasmid or virus) that can be conveniently subjected to recombinant DNA procedures and can bring about expression of the polynucleotide. The choice of the vector will typically depend on the compatibility of the vector with the host cell into which the vector is to be introduced. The vector may be a linear or closed circular plasmid.

The vector may be an autonomously replicating vector, i.e., a vector that exists as an extra-chromosomal entity, the replication of which is independent of chromosomal replication, e.g., a plasmid, an extra-chromosomal element, a mini-chromosome, or an artificial chromosome. The vector may contain any means for assuring self-replication. Alternatively, the vector may be one that, when introduced into the host cell, is integrated into the genome and replicated together with the chromosome(s) into which it has been integrated. Furthermore, a single vector or plasmid or two or more vectors or plasmids that together contain the total DNA to be introduced into the genome of the host cell, or a transposon, may be used.

The vector preferably contains one or more selectable markers that permit easy selection of transformed, transfected, transduced, or the like cells. A selectable marker is a gene the product of which provides for biocide or viral resistance, resistance to heavy metals, prototrophy to auxotrophy, and the like.

Examples of bacterial selectable markers are *Bacillus licheniformis* or *Bacillus subtilis* genes or markers that confer antibiotic resistance such as ampicillin, chloramphenicol, kanamycin, neomycin, spectinomycin, or tetracycline resistance. Suitable markers for yeast host cells include, but are not limited to, ADE2, HIS3, LEU2, LYS2, MET3, TRP1, and URA3. Selectable markers for use in a filamentous fungal host cell include, but are not limited to, amdS (acetamidase), argB (ornithine carbamoyltransferase), bar (phosphinothricin acetyltransferase), hph (hygromycin phosphotransferase), niaD (nitrate reductase), pyrG (orotidine-5'-phosphate decarboxylase), sC (sulfate adenyltransferase), and trpC (anthranilate synthase), as well as equivalents thereof. Preferred for use in an *Aspergillus* cell are *Aspergillus nidulans* or *Aspergillus oryzae* amdS and pyrG genes and a *Streptomyces hygroscopicus* gene.

The vector preferably contains an element(s) that permits integration of the vector into the host cell's genome or autonomous replication of the vector in the cell independent of the genome.

When integration into the host cell genome occurs, integration of the sequences into the genome may rely on homologous or non-homologous recombination. Alternatively, the vector may contain additional polynucleotides for directing integration by homologous recombination into the genome of the host cell at a precise location(s) in the chromosome(s). To increase the likelihood of integration at a precise location, the integrational elements should contain a sufficient number of nucleic acids, such as 100 to 10,000 base pairs, 400 to 10,000 base pairs, and 800 to 10,000 base pairs, which have a high degree of sequence identity to the corresponding target sequence to enhance the probability of homologous recombination. The integrational elements may be any sequence that is homologous with the target sequence in the genome of the host cell. Furthermore, the integrational elements may be non-encoding or encoding polynucleotides. On the other hand, the vector may be integrated into the genome of the host cell by non-homologous recombination.

For autonomous replication, the vector may further comprise an origin of replication enabling the vector to replicate autonomously in the host cell in question. The origin of replication may be any plasmid replicator mediating autonomous replication that functions in a cell. The term "origin of replication" or "plasmid replicator" means a polynucleotide that enables a plasmid or vector to replicate in vivo. Examples of bacterial origins of replication are the origins of replication of plasmids pBR322, pUC19, pACYC177, and pACYC184 permitting replication in *E. coli,* and pUB1 10, pE194, pTA1060, and pAMβ1 permitting replication in Bacillus. Examples of origins of replication for use in a yeast host cell are the 2 micron origin of replication, ARS1, ARS4, the combination of ARS1 and CEN3, and the combination of ARS4 and CEN6. Examples of origins of replication useful in a filamentous fungal cell are AMA1 and ANSI (Gems et al., 1991, Gene 98: 61 -67; Cullen et al., 1987, Nucleic Acids Res. 15: 9163-9175; WO 00/24883). Isolation of the AMA1 gene and construction of plasmids or vectors comprising the gene can be accomplished according to the methods disclosed in WO 00/24883.

More than one copy of a polynucleotide of the present disclosure may be inserted into a host cell to increase production of a polypeptide. An increase in the copy number of the polynucleotide can be obtained by integrating at least one additional copy of the sequence into the host cell genome or by including an amplifiable selectable marker gene with the polynucleotide where cells containing amplified copies of the selectable marker gene, and thereby additional copies of the polynucleotide, can be selected for by cultivating the cells in the presence of the appropriate selectable agent.

The procedures used to ligate the elements described above to construct the recombinant expression vectors of the present disclosure are well known to one skilled in the art (see, e.g., Sambrook et al., 1989, supra).

### Host cells

The present disclosure also relates to recombinant host cells comprising a polynucleotide encoding an epimerase according to the present disclosure operably linked to one or more control sequences that direct the production of the epimerase of the present disclosure. A construct or vector comprising a polynucleotide encoding an epimerase according to the present disclosure is introduced into a host cell so that the construct or vector is maintained as a chromosomal integrant or as a self-replicating extra-chromosomal vector as described earlier.

The present disclosure further relates to a recombinant host cell that further comprises a polynucleotide encoding a peptide according to the present disclosure operably linked to one or more control sequences that direct the production of the peptide. In particular, the host cell can co-express the epimerase and the peptide according to the present disclosure. In this aspect, the host cell both produces a peptide of the present disclosure and the epimerase which is able to modify the antimicrobial peptide by changing the configuration of peptide's amino acid from a L-configuration to a D-configuration.

In a preferred aspect, the host cell comprises a nucleic acid encoding an epimerase of the present disclosure heterologous to the host cell. In an alternative preferred aspect, the host cell comprises a nucleic acid encoding a peptide heterologous to the host cell. In another preferred aspect, the host cell comprises nucleic acids encoding a peptide and an epimerase, both heterologous to the host cell.

The term "host cell" encompasses any progeny of a parent cell that is not identical to the parent cell due to mutations that occur during replication. The choice of a host cell will to a large extent depend upon the gene encoding the polypeptide and its source.

The host cell may be any cell useful in the recombinant production of an epimerase of the present disclosure, e.g., a prokaryote or a eukaryote.

The prokaryotic host cell may be any Gram-positive or Gram-negative bacterium. Gram-positive bacteria include, but are not limited to, *Bacillus, Clostridium, Enterococcus, Geobacillus, Lactobacillus, Lactococcus, Oceanobacillus, Staphylococcus, Streptococcus,* and *Streptomyces.* Gram-negative bacteria include, but are not limited to, *Campylobacter, E. coli, Flavobacterium, Fusobacterium, Helicobacter, Ilyobacter, Neisseria, Pseudomonas, Salmonella,* and *Ureaplasma.* The bacterial host cell may be any *Bacillus* cell including, but not limited to, *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* and *Bacillus thuringiensis* cells. The bacterial host cell may also be any *Streptococcus* cell including, but not limited to, *Streptococcus equisimilis, Streptococcus pyogenes, Streptococcus uberis, Streptococcus equi* and *Streptococcus zooepidemicus* cells. The bacterial host cell may further be any *Streptomyces* cell including, but not limited to, *Streptomyces achromogenes, Streptomyces avermitilis, Streptomyces coelicolor, Streptomyces griseus,* and *Streptomyces lividans* cells.

The introduction of DNA into a *Bacillus* cell may be effected by protoplast transformation (see, e.g., Chang and Cohen, 1979, Mol. Gen. Genet. 168: 1 11 -1 15), competent cell transformation (see, e.g., Young and Spizizen, 1961, J. Bacteriol. 81: 823-829, or Dubnau and Davidoff-Abelson, 1971, J. Mol. Biol. 56: 209-221), electroporation (see, e.g., Shigekawa and Dower, 1988, Biotechniques 6: 742-751), or conjugation (see, e.g., Koehler and Thorne, 1987, J. Bacteriol. 169: 5271 -5278). The introduction of DNA into an *E. coli* cell may be effected by protoplast transformation (see, e.g., Hanahan, 1983, J. Mol. Biol. 166: 557-580) or electroporation (see, e.g., Dower et al, 1988, Nucleic Acids Res. 16: 6127-6145). The introduction of DNA into a *Streptomyces* cell may be effected by protoplast transformation, electroporation (see, e.g., Gong et al., 2004, Folia Microbiol. (Praha) 49: 399-405), conjugation (see, e.g., Mazodier ei a/., 1989, J. Bacteriol. 171: 3583-3585), or transduction (see, e.g., Burke et al., 2001, Proc. Natl. Acad. Sci. USA 98: 6289-6294). The introduction of DNA into a *Pseudomonas* cell may be effected by electroporation (see, e.g., Choi et al., 2006, J. Microbiol. Methods 64: 391 -397) or conjugation (see, e.g., Pinedo and Smets, 2005, Appl. Environ. Microbiol. 71: 51 -57). The introduction of DNA into a *Streptococcus* cell may be effected by natural competence (see, e.g., Perry and Kuramitsu, 1981, Infect. Immun. 32: 1295-1297), protoplast transformation (see, e.g., Catt and Jollick, 1991, Microbios 68: 189-207), electroporation (see, e.g., Buckley et al., 1999, Appl. Environ. Microbiol. 65: 3800-3804), or conjugation (see, e.g., Clewell, 1981, Microbiol. Rev. 45: 409-436). However, any method known in the art for introducing DNA into a host cell can be used.

The host cell may also be a eukaryote, such as a mammalian, insect, plant, or fungal cell. The host cell may be a fungal cell. "Fungi" as used herein includes the phyla *Ascomycota, Basidiomycota, Chytridiomycota,* and *Zygomycota* as well as the *Oomycota* and all mitosporic fungi (as defined by Hawksworth et al., In, Ainsworth and Bisby's Dictionary of The Fungi, 8th edition, 1995, CAB International, University Press, Cambridge, UK). The fungal host cell may be a yeast cell. "Yeast" as used herein includes ascosporogenous yeast (Endomycetales), basidiosporogenous yeast, and yeast belonging to the *Fungi imperfecti* (Blastomycetes). Since the classification of yeast may change in the future, for the purposes of this disclosure, yeast shall be defined as described in Biology and Activities of Yeast (Skinner, Passmore, and Davenport, editors, Soc. App. Bacteriol. Symposium Series No. 9, 1980). The yeast host cell may be a *Candida, Hansenula, Kluyveromyces, Pichia, Saccharomyces, Schizosaccharomyces,* or *Yarrowia* cell, such as a *Kluyveromyces lactis, Saccharomyces carlsbergensis, Saccharomyces cerevisiae, Saccharomyces diastaticus, Saccharomyces douglasii, Saccharomyces kluyveri, Saccharomyces norbensis, Saccharomyces oviformis,* or *Yarrowia lipolytica* cell. The fungal host cell may be a filamentous fungal cell. "Filamentous fungi" include all filamentous forms of the subdivision *Eumycota* and *Oomycota* (as defined by Hawksworth et al., 1995, supra). The filamentous fungi are generally characterized by a mycelial wall composed of chitin, cellulose, glucan, chitosan, mannan, and other complex polysaccharides. The filamentous fungal host cell may be an *Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes,* or *Trichoderma* cell. For example, the filamentous fungal host cell may be an *Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Thielavia terrestris, Trametes villosa, Trametes versicolor, Trichoderma harzianum, Trichoderma koningii, Trichoderma longibrachiatum, Trichoderma reesei,* or *Trichoderma viride* cell.

Fungal cells may be transformed by a process involving protoplast formation, transformation of the protoplasts, and regeneration of the cell wall in a manner known *per se.* Suitable procedures for transformation of *Aspergillus* and *Trichoderma* host cells are described in EP 238023, Yelton et al., 1984, Proc. Natl. Acad. Sci. USA 81: 1470-1474, and Christensen et al., 1988, Bio/Technology 6: 1419-1422. Suitable methods for transforming *Fusarium* species are described by Malardier et al., 1989, Gene 78: 147-156, and WO 96/00787. Yeast may be transformed using the procedures described by Becker and Guarente, In Abelson, J.N. and Simon, M.I., editors, Guide to Yeast Genetics and Molecular Biology, Methods in Enzymology, Volume 194, pp 182-187, Academic Press, Inc., New York; Ito et al., 1983, J. Bacteriol. 153: 163; and Hinnen et al., 1978, Proc. Natl. Acad. Sci. USA 75: 1920.

The cell can also be a mammalian cell, for example COS, CHO (US 4,889,803; US 5,047,335). In a particular aspect, the cell is non-human and non-embryonic. In addition, the epimerase of the disclosure could be produce by a non-human transgenic animal, for instance in the milk produces by the animal.

The cell can be a plant cell. Then, the epimerase of the disclosure could be produced by a transgenic plant.

Alternatively, it is also provided a method for producing an epimerase according to the present disclosure, comprising culturing the host cell as defined above, under conditions conducive to the production of the epimerase, and recovering and/or purifying the epimerase. Alternatively, it is also provided a method for producing an epimerase according to the present disclosure, comprising the *in vitro* expression of the epimerase with a nucleic acid encoding the epimerase as defined above. Optionally, the method further comprises a step of immobilizing the epimerase on a solid support.

The enzyme may be recovered using methods known in the art. For example, the enzyme may be recovered from the nutrient medium by conventional procedures including, but not limited to, collection, centrifugation, filtration, extraction, spray-drying, evaporation, or precipitation.

The enzyme may be purified by a variety of procedures known in the art including, but not limited to, chromatography (e.g., ion exchange, affinity, hydrophobic, chromatofocusing, and size exclusion), electrophoretic procedures (e.g., preparative isoelectric focusing), differential solubility (e.g., ammonium sulfate precipitation), SDS-PAGE, or extraction (see, e.g., Protein Purification, Janson and Ryden, editors, VCH Publishers, New York, 1989) to obtain substantially pure polypeptides. In an alternative aspect, the enzyme is not recovered, but rather a host cell of the present disclosure expressing the enzyme is used as a source of the enzyme.

It is also provided a composition or a kit comprising the isolated or recombinant epimerase as defined above. In particular, the composition or kit may include iron (e.g., (NH₄)₂Fe(SO₄)₂) and sulfur (e.g., Na₂S) as enzyme additives and a reducing agent such as dithiothreitol or betamercaptoethanol. In addition the composition may include S-adenosyl L-methionine (SAM), the enzyme cofactor or methionine and ATP when in the presence of SAM synthase. Optionally the epimerase can be immobilized on a solid support.

The present disclosure also relates to the use of an epimerase as defined above, a composition, kit or solid support comprising the epimerase, or a recombinant host cell comprising a nucleic acid, a recombinant nucleic acid construct or a recombinant vector comprising a nucleic acid sequence encoding the epimerase as defined above, for producing peptide with D-configured amino acids.

### Use of the epimerase for converting L-amino acid into D-amino acid in a peptide

Accordingly, the present disclosure relates to the use of an epimerase of the disclosure for converting L-amino acid into D-amino acid in a peptide. It also relates to a method for converting L-amino acid into D-amino acid in a peptide comprising contacting an epimerase of the present disclosure with the peptide, and optionally recovering the peptide with at least one amino acid converted from a L-configuration to a D-configuration.

In a preferred aspect, the present disclosure relates to a method for preparing a peptide according to the present disclosure, wherein the method comprises a step of contacting a peptide according to the present disclosure with an epimerase as defined above. It also relates to the use of an epimerase as defined above for preparing a peptide according to the present disclosure. In particular, the peptide can have any particular sequence as defined above. Preferably, the method is carried out *in vitro.* In a first aspect, the peptide comprises a sequence of 17 residues having at least 40 % of identity with the sequence in positions 33 to 49 of SEQ ID No 1 and said sequence comprising the sequences W-Y-F-[V/I/A] (SEQ ID No 71) and W-[I/V/A]-X4-G-S (SEQ ID No 69), wherein X4 is any amino acid, and a connecting sequence between SEQ ID No 69 and SEQ ID No 71 of 4-8 amino acids. In a second aspect, the peptide comprises a sequence of 24 residues having at least 40 % of identity with the sequence in positions 26 to 49 of SEQ ID No 1 and comprising the sequences N/D-D-L-W-Y-F-[V/I/A] (SEQ ID No 21) and W-I/V-X4-G-S (SEQ ID No 22), wherein X4 is any amino acid.

Finally, the present disclosure relates to a method for preparing a peptide according to the present disclosure, wherein the method comprises culturing a recombinant host cell according to the present disclosure in conditions suitable for the co-expression the peptide and the epimerase and recovering the peptide having at least one D-configured amino acid. Preferably, the method is carried out ex *vivo.* It also relates to the use of a recombinant host cell according to the present disclosure for preparing a peptide as defined above.

In a particular aspect, the sources of epimerase and peptide can be matched. For instance, an epimerase from *Bacillus subtilis* could be preferably used for preparing a peptide from or derived from *Bacillus subtilis.* In this particular aspect, if the peptide as defined above comprises a sequence having at least 80, 85, 90 or 95 % of identity with a sequence selected among SEQ ID Nos 1-5 or a fragment thereof of at least 24 consecutive residues, then the chosen epimerase will have at least 80, 85, 90 or 95 % of identity with a sequence selected among SEQ ID Nos 25-35. If the peptide as defined above comprises a sequence having at least 80, 85, 90 or 95 % of identity with a sequence selected among SEQ ID Nos 6-9 or a fragment thereof of at least 24 consecutive residues, then the chosen epimerase will have at least 80, 85, 90 or 95 % of identity with a sequence selected among SEQ ID Nos 36-38. If the peptide as defined above comprises a sequence having at least 80, 85, 90 or 95 % of identity with a sequence of SEQ ID No 10 or a fragment thereof of at least 24 consecutive residues, then the chosen epimerase will have at least 80, 85, 90 or 95 % of identity with a sequence selected among SEQ ID Nos 39-40. If the peptide as defined above comprises a sequence having at least 80, 85, 90 or 95 % of identity with a sequence selected among SEQ ID Nos 11-13 and 17-18 or a fragment thereof of at least 24 consecutive residues, then the chosen epimerase will have at least 80, 85, 90 or 95 % of identity with a sequence selected among SEQ ID Nos 41-50. If the peptide as defined above comprises a sequence having at least 80, 85, 90 or 95 % of identity with a sequence selected among SEQ ID Nos 14-16 or a fragment thereof of at least 24 consecutive residues, then the chosen epimerase will have at least 80, 85, 90 or 95 % of identity with a sequence of SEQ ID No 51. If the peptide as defined above comprises a sequence having at least 80, 85, 90 or 95 % of identity with a sequence of SEQ ID No 19 or a fragment thereof of at least 24 consecutive residues, then the chosen epimerase will have at least 80, 85, 90 or 95 % of identity with a sequence of SEQ ID No 52.

### Definitions

*About:* When used herein, "about" means more or less 10 %, preferably more or less 5 %. For instance, about 100 means between 90 and 110, preferably between 95 and 105.

*"consists of," "consists essentially of" or "substantially comprises"*: The description herein of any aspect aspect of the disclosure using terms such as reference to an element or elements is intended to provide support for a similar aspect aspect of the disclosure that "consists of'," "consists essentially of" or "substantially comprises" that particular element or elements, unless otherwise stated or clearly contradicted by context. For instance, a peptide or protein described herein as comprising a particular sequence should be understood as also describing a peptide or protein consisting of that sequence, unless otherwise stated or clearly contradicted by context. By "consists essentially of" is intended that the peptide or protein consists of that sequence, but it may also include 1, 2, 3, 4, 5, 6, 7, 8, 9 or 10 substitutions, additions or deletions. In particular, by "essentially consist in", it may be intended that the peptide may include 1, 2, 3, 4, 5, 6, 7, 8, 9 or 10 additional amino acids at the N or C-terminal end and 1, 2 or 3 substitutions, deletions or additions.

*Coding sequence*: The term "coding sequence" means a polynucleotide, which directly specifies the amino acid sequence of a polypeptide. The boundaries of the coding sequence are generally determined by an open reading frame, which begins with a start codon such as ATG, GTG, or TTG and ends with a stop codon such as TAA, TAG, or TGA. The coding sequence may be a genomic DNA, cDNA, synthetic DNA, or a combination thereof.

*Control sequences*: The term "control sequences" means nucleic acid sequences necessary for expression of a polynucleotide encoding an epimerase of the present disclosure. Control sequences may be native (i.e., from the same gene) or heterologous (i.e., from a different gene and/or a different species) to the polynucleotide encoding the epimerase. Preferably, control sequences are heterologous. Well-known control sequences and currently used by the person skilled in the art will be preferred. Such control sequences include, but are not limited to, a leader, polyadenylation sequence, propeptide sequence, promoter, signal peptide sequence, and transcription terminator. At a minimum, the control sequences include a promoter, and transcriptional and translational stop signals. The control sequences may be provided with linkers for the purpose of introducing specific restriction sites facilitating ligation of the control sequences with the coding region of the polynucleotide encoding the epimerase. The functional combination of control sequences and coding sequences can be referred as *expression cassette.*

*Expression*: The term "expression" includes any step involved in the production of a polypeptide including, but not limited to, transcription, post-transcriptional modification, translation, post-translational modification, and secretion.

*Expression vector*: The term "expression vector" means a linear or circular DNA molecule that comprises a polynucleotide encoding the epimerase of the disclosure and is operably linked to control sequences that provide for its expression. Then the expression vector comprises an expression cassette suitable for expressing the epimerase of the disclosure.

*Isolated*: The term "isolated" means a substance in a form or environment that does not occur in nature. Non-limiting examples of isolated substances include (1) any non-naturally occurring substance, (2) any substance including, but not limited to, any enzyme, variant, nucleic acid, protein, peptide or cofactor, that is at least partially removed from one or more or all of the naturally occurring constituents with which it is associated in nature; (3) any substance modified by the hand of man relative to that substance found in nature; or (4) any substance modified by increasing the amount of the substance relative to other components with which it is naturally associated (e.g., multiple copies of a gene encoding the substance; use of a stronger promoter than the promoter naturally associated with the gene encoding the substance).

*Recombinant:* Recombinant refers to a nucleic acid construct, a vector and a protein produced by genetic engineering.

*Heterologous:* in the context of a host cell, a vector or a nucleic acid construct, it designates a coding sequence for the epimerase/peptide introduced into the host cell, the vector or the nucleic acid construct by genetic engineering. In the context of a host cell, it can mean that the coding sequence for the epimerase/peptide originates from a source different from the cell in which it is introduced. For instance, an epimerase from *Bacillus subtilis* is expressed in *E. coli.* Alternatively, it can also mean that the coding sequence for the epimerase/peptide comes from the same species as the cell in which it is introduced but it is considered heterologous due to its environment which is not natural, for example because it is under the control of a promoter which is not its natural promoter, or is introduced at a location which differs from its natural location.

*Nucleic acid construct*: The term "nucleic acid construct" means a nucleic acid molecule, either single- or double-stranded, which is modified to contain segments of nucleic acids in a manner that would not otherwise exist in nature or which is synthetic, which comprises one or more control sequences.

*Operably linked*: The term "operably linked" means a configuration in which a control sequence is placed at an appropriate position relative to a coding sequence, in such a way that the control sequence directs expression of the coding sequence.

*Sequence identity*: The sequence identity between two amino acid sequences is described by the parameter "sequence identity". For purposes of the present disclosure, the "percentage identity" between two amino acid sequences (A) and (B) is determined by comparing the two sequences aligned in an optimal manner, through a window of comparison. Said alignment of sequences can be carried out by well-known methods, for example, using the algorithm for global alignment of Needleman-Wunsch. Protein analysis software matches similar sequences using measures of similarity assigned to various substitutions, deletions and other modifications, including conservative amino acid substitutions. Once the total alignment is obtained, the percentage of identity can be obtained by dividing the full number of identical amino acid residues aligned by the full number of residues contained in the longest sequence between the sequence (A) and (B).

Sequence identity is typically determined using sequence analysis software. For comparing two amino acid sequences, one can use, for example, the tool "Emboss needle" for pairwise sequence alignment of proteins providing by EMBL-EBI and available on:
www.ebi.ac.uk/Tools/services/web/toolform.ebi?tool=emboss needle&context=protein,
using default settings : (I) Matrix : BLOSUM62, (ii) Gap open : 10, (iii) gap extend : 0.5, (iv) output format: pair, (v) end gap penalty : false, (vi) end gap open : 10, (vii) end gap extend : 0.5.

Alternatively, Sequence identity can also be typically determined using sequence analysis software Clustal Omega using the HHalign algorithm and its default settings as its core alignment engine. The algorithm is described in Söding, J. (2005) 'Protein homology detection by HMM-HMM comparison'. Bioinformatics 21, 951-960, with the default settings.

*Amino acids*: The amino acid sequences defined herein are represented by a one-letter symbol as shown below: A, Ala, (alanine); R, Arg, (arginine); N , Asn, (asparagine); D, Asp, (aspartic acid); C, Cys, (cysteine); Q, Gln, (glutamine); E, Glu, (glutamic acid); G , Gly, (glycine); H , His, (histidine); I , Ile, (isoleucine); L, Leu, (leucine); K, Lys, (lysine); M , Met, (methionine); F, Phe, (phenylalanine); P , Pro, (proline); S , Ser, (serine); T, Thr, (threonine); W, Trp, (tryptophan); Y, Tyr, (tyrosine); and V , Val, (valine).

*Conserved*: By conserved amino acid is intended that a defined sequence is aligned with the reference sequence and the residue of the defined sequence corresponding the position indicated in the reference sequence is identical to the residue present in the reference sequence. The alignment can be performed by any available method, and in particular by the method disclosed for identity determination just above, more preferably by Clustal Omega. The residue position is indicated in the reference sequence.

The term "antimicrobial" as employed herein refers to an antibacterial, antiviral, antifungal and/or antiparasitic activity. Said activity may be evaluated by measuring different parameters such as IC₅₀ or MIC.

"IC₅₀" or "half maximal inhibitory concentration" is the concentration of a substance needed to reduce the growth *in vitro* of a population of microorganisms by half.

"MIC" or "minimum inhibitory concentration" is the lowest concentration of a substance that will totally inhibit microbial growth after 18 hours of incubation, generally at 37°C, in the presence of said substance.

The term "lethal concentration, 50%" or "LC₅₀" as employed herein refers to the concentration of substance required to kill half a population. LC₅₀ is a quantitative indicator of the toxicity of a substance. In particular, LC₅₀ is employed herein to evaluate the cytolytic activity of AMP and in this case corresponds to the concentration of peptide inducing lysis of half the cell population.

### BRIEF DESCRIPTION OF THE DRAWINGS

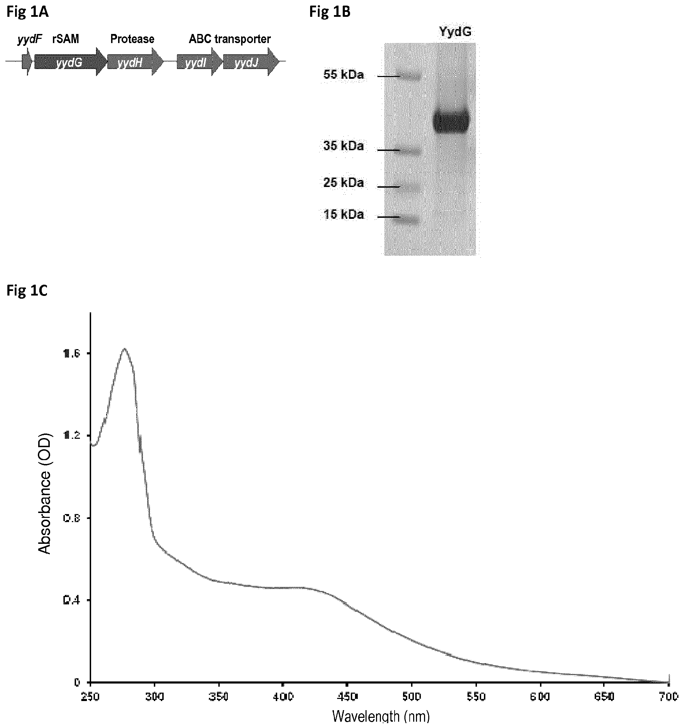

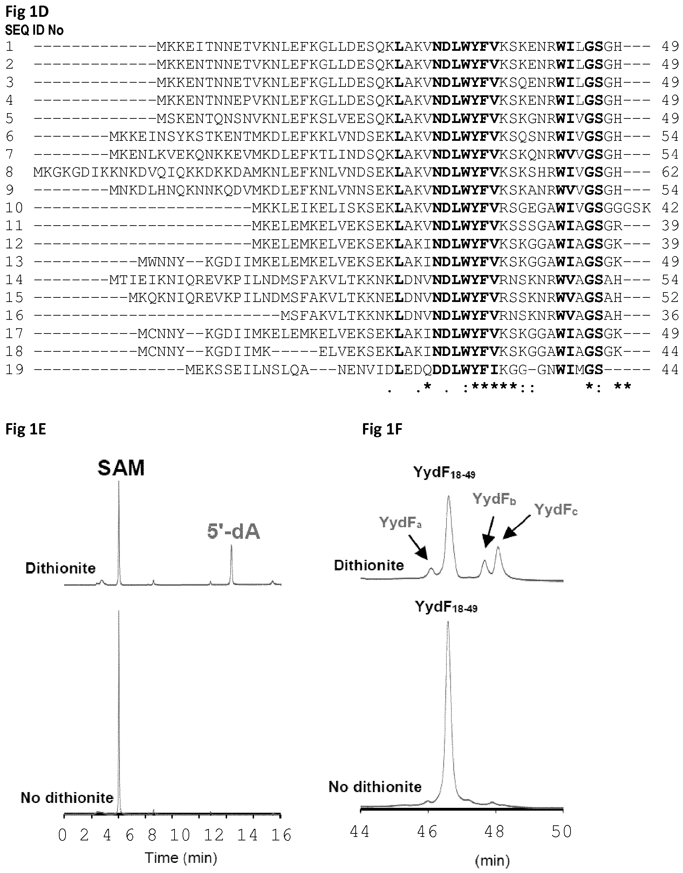

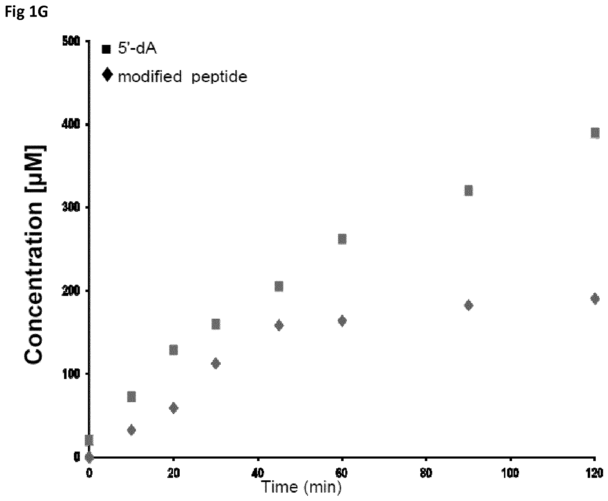

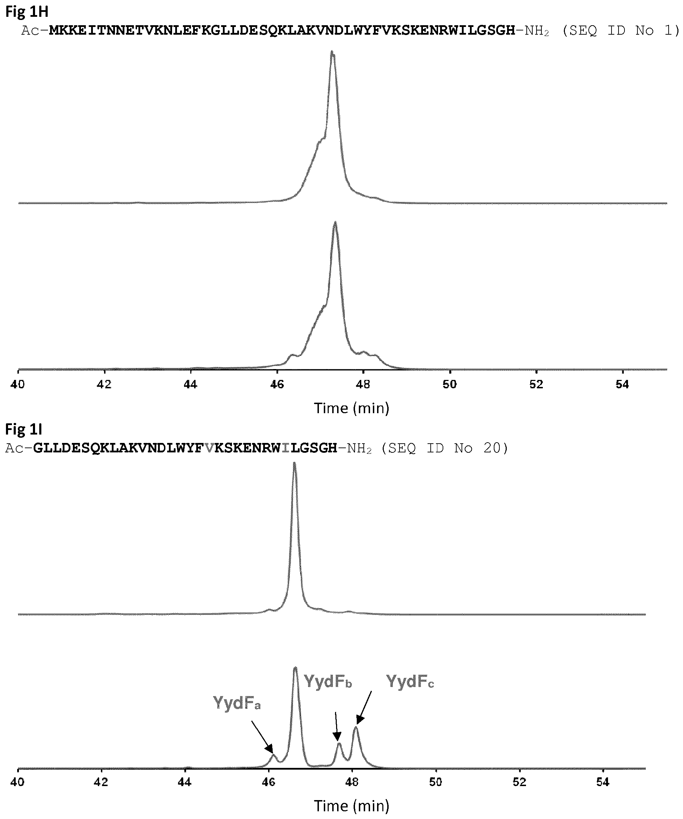

**FIGURE 1****. Purification, spectroscopic analysis and activity of YydG.** (**Fig 1A**) Structure of the yydFGHIJ operon. *yydF*: Putative peptide, *yydG*: radical SAM enzyme, *yydH*: protease, *yydIJ* ABC-type transporter. (**Fig 1B**) Gel electrophoresis analysis of purified YydG expressed in *E. coli.* (**Fig 1C**) UV-visible spectrum of anaerobically reconstituted YydG. (**Fig 1D**) Multiple alignments of close YydF homologs found in particular in *B. subtilis* and *Staphylococci* species.. (**Fig 1E**) HPLC analysis of SAM cleavage (257 nm) and (**Fig 1F**) peptides produced in reaction (280 nm) after 4 hours incubation under anaerobic conditions in the presence or the absence of sodium dithionite (2 mM). (**Fig 1G**) Time course for the production of 5'-dA (■) and modified peptides (◆) quantified by reverse phase HPLC and monitored by UV-visible detection (280 nm). YydG (100µM) was incubated under anaerobic conditions with sodium dithionite (2 mM) in the presence of 1 mM of substrate YydF₁₈₋₄₉. **(****Fig 1H** **& I)** HPLC analysis of YydG incubated with (Fig 1H) YydF or (Fig 1I) a truncated version of YydF18-49. YydG, after anaerobic reconstitution, was incubated under anaerobic conditions in the presence of DTT (6 mM) and SAM (1 mM) with or without sodium dithionite (2 mM).

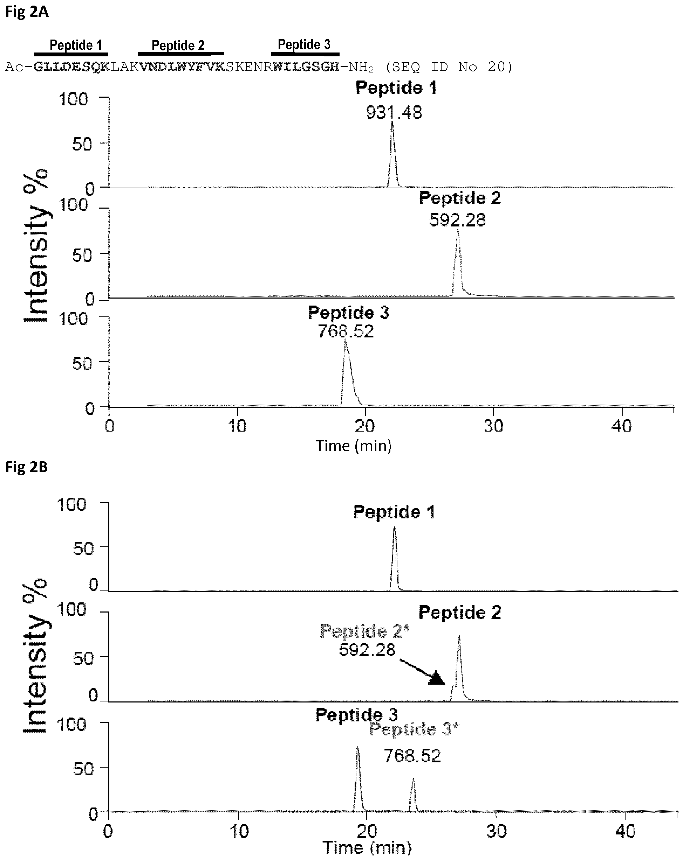

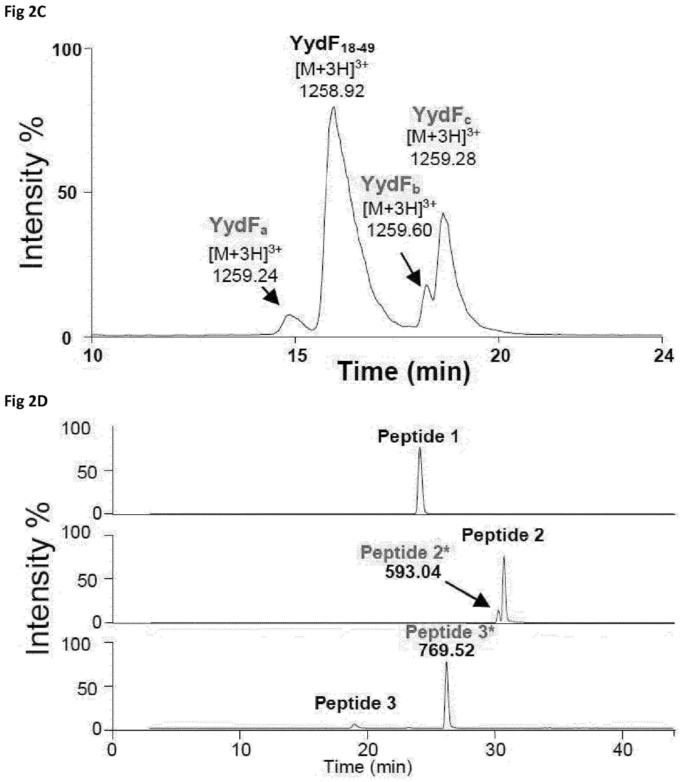

**Figure 2** - **YydG catalyzes H-atom transfer to the peptide backbone.** Tryptic peptide mapping and LC-MS analysis of (**Fig 2A**) YydF₁₈₋₄₉ or (**Fig 2B**) YydF₁₈₋₄₉ after incubation with YydG. Numbers indicate the m/z value for each peptide. Sequence in bold indicates the relevant peptide identified by LC-MS (*i*.*e*. **Peptide 1**: NH₂-GLLDESQK, [M+H]⁺= 931.48 ; **Peptide 2**: VNDLWYF [M+2H]²⁺=592.31 ; **Peptide 3:** WILGSGH-Ac, [M+H]⁺ =768.41). (**Fig 2C**) LC-MS analysis of the peptide YydF₁₈₋₄₉ after incubation with YydG in deuterated buffer. (**Fig 2D**) Tryptic peptide mapping and LC-MS analysis of YydF₁₈₋₄₉ after incubation with YydG in deuterated buffer.

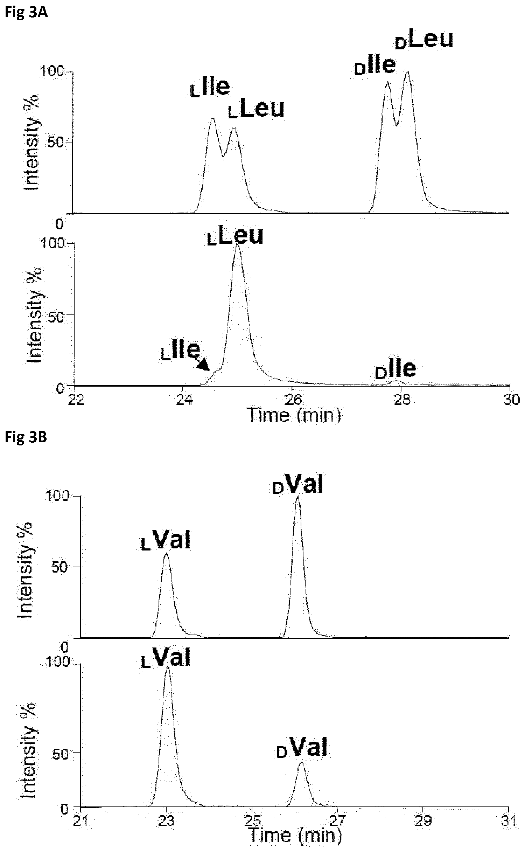

**Figure 3** - **YydG catalyzes amino acid epimerization.** LC-MS/MS analysis of a (**Fig 3A**) L-Ile/D-allo-Ile and (**Fig 3B**) L-/D-Val (upper traces) compared with the amino acids obtained after incubation of YydF₁₈₋₄₉ with the rSAM enzyme YydG in deuterated buffer (lower traces). The amino acids were derivatized by *N*-α-(2,4-dinitro-5-fluorophenyl)-L-valinamide (L-FDVA) and detected by LC-MS as FDVA-derivatives.

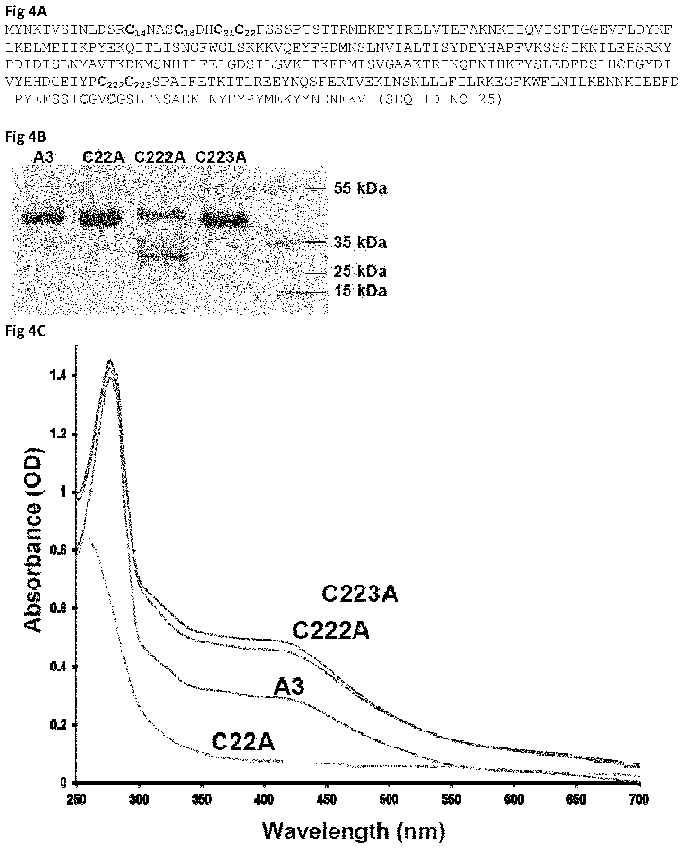

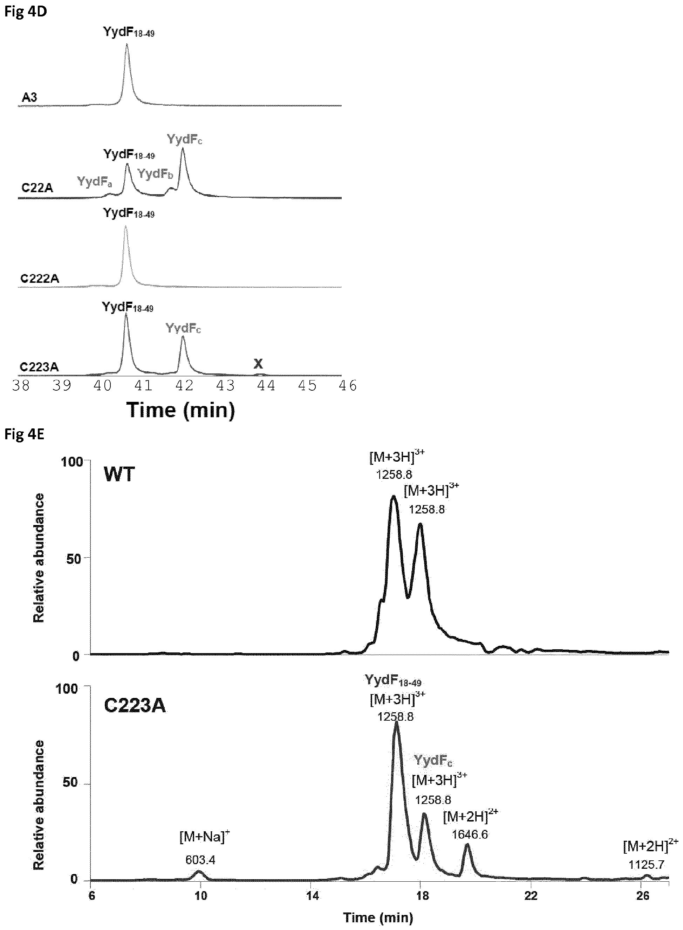

**Figure 4** - **Activity of YydG mutants.** (**Fig 4A**) Sequence of YydG with cysteine residues highlighted. (**Fig 4B**) Gel electrophoresis analysis of the purified mutant enzymes. (**Fig 4C**) UV-visible spectra of A3 (i.e. AxxxAxxA) (blue trace), C22A (green trace), C222A (red trace) and C223A (purple trace) mutants after anaerobic reconstitution. (**Fig 4D**) HPLC analysis of the reaction after incubation of YydF18-49 and the four mutants in the presence of SAM (1 mM) and sodium dithionite. (**Fig 4E**) LC-MS analysis of the peptides produced by the C223A mutant and their corresponding masses and sequences.

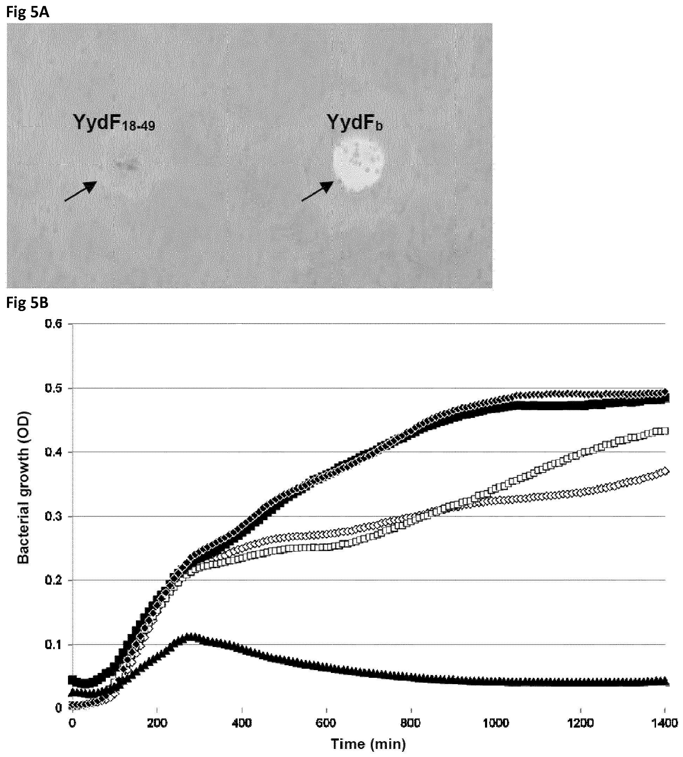

**Figure 5** - **Activity of YydF18-49 of YydG.** (**Fig 5A**) Plate growth inhibition assay of B. subtilis in the presence of YydF₁₈₋₄₉ or the epimierized product YydFb. (**Fig 5B**) Growth of *Bacillus subtilis* in LB medium in the absence of peptide (0) or in the presence of the YydF₁₈₋₄₉ peptide with a free C-terminus: Ac-GLLDESQKLAKVNDLWYFVKSKENRWILGSGH (SEQ ID No 20) containing either no modification (■), a D-allo-Ile (◆), a D-Val (◇) or two-epimerized residues: Ac-GLLDESQKLAKVNDLWYF{d-V}KSKENRW{d-I}LGSGH (SEQ ID No 20) (▲). The OD values are the means of 3 independent cultures.
**Figure 6****-** Multiple alignments of close YydG homologs found in particular in *B. subtilis* and *Staphylococci* species.

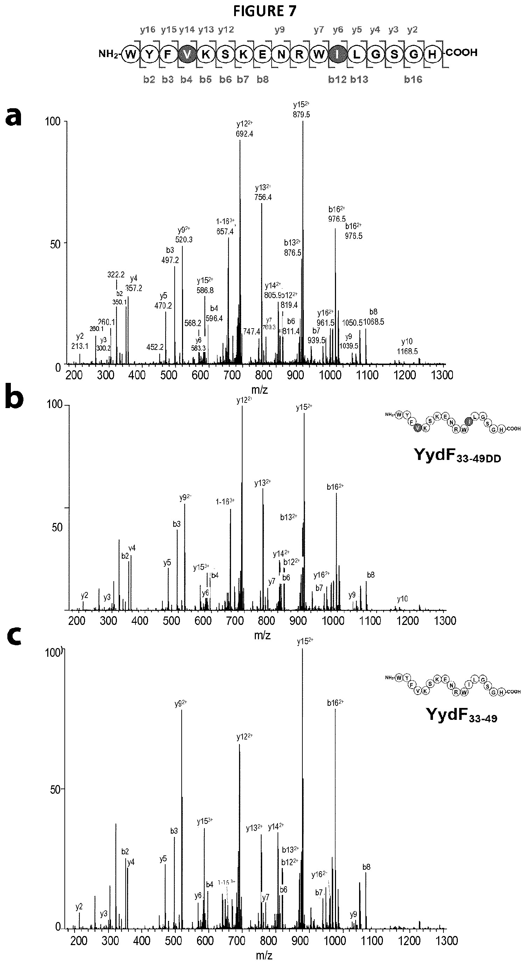

**Figure 7** - **Mass spectrometry analysis of the YydF peptide isolated from *B. subtilis***and named YydF₃₃₋₄₉. **LC-MS/MS analysis of (a) the peptide secreted by *B*. *subtilis,* (b) synthetic YydF_{33-49DD} peptide containing two D-amino acids residues and (c) synthetic YydF₃₃₋₄₉ peptide.** Relevant ions are indicated. *Tables 1 for full assignment.* In the peptide sequence, amino acids with a D-configuration are indicated in grey.

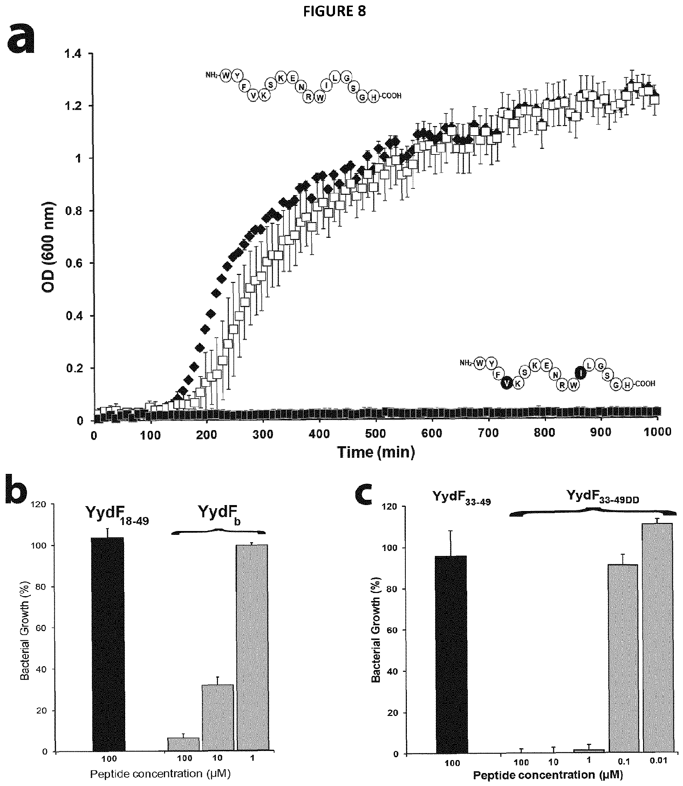

**Figure 8** **- (A) Growth of** *B.* ***subtilis* in liquid LB medium in the presence of YydF₃₃₋₄₉ or YydF₃₃₋₄₉DD.** *B. subtilis* was grown in LB medium alone (◆), in the presence of **YydF₃₃₋₄₉** (□) or **YydF_{33-49DD}** (■). Each measurement is the mean of three growth experiments with the SD indicated. The epimerized residues are in black. **(b) Growth ratio of** *B.* ***subtilis* in presence of YydF₁₈₋₄₉ (100 µM) or YydF_{18-49DD}** (100, 10 or 1 µM). Ratios were determined by comparison with growth in the absence of peptide. **(c) Growth ratio of** *B.* ***subtilis* in presence of YydF₃₃₋₄₉ or YydF_{33-49DD} (100, 10, 1, 0.1 or 0.01 µM).** Ratios were determined by comparison with bacterial growth in the absence of peptide.

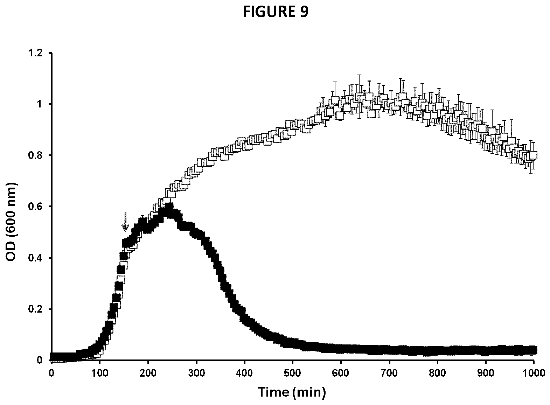

**Figure 9** - Growth of B. subtilis in liquid LB medium in the presence of **YydF₁₈₋₄₉** (□) or after addition (arrow) of the YydF_{18-49DD} (■) after 3 hours of growth. Each measurement is the mean of three growth experiments with the SD indicated.

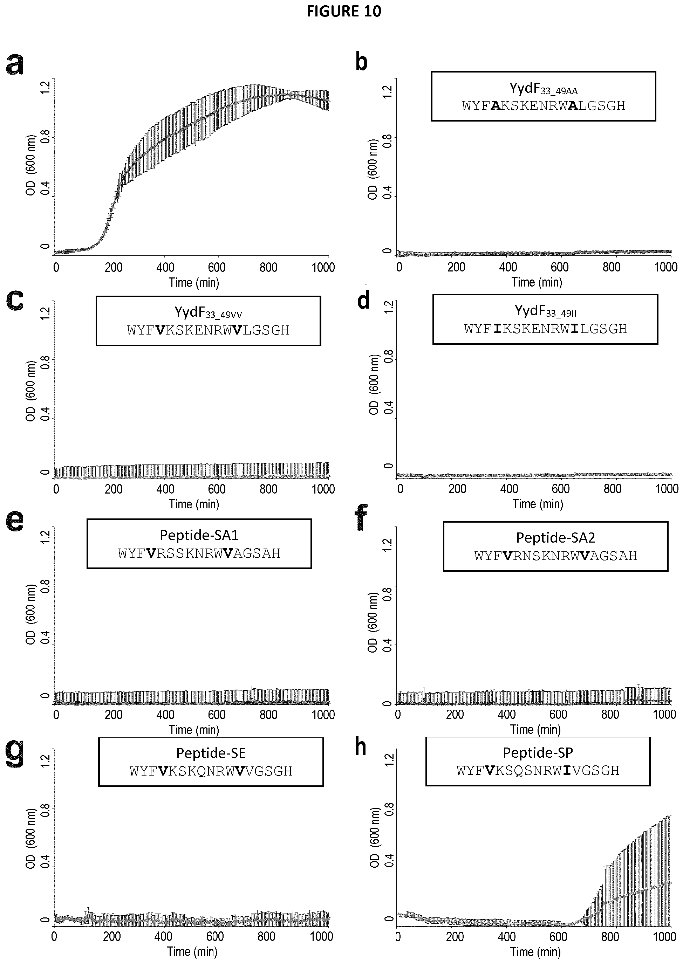

**Figure 10** - **Growth of** *B.* ***subtilis* in liquid LB medium in the presence of YydF₃₃₋₄₉ (a), YydF**_{**33**_}**_{49AA} (b), YydF_{33_49VV} (c), YydF_{33_49II} (d), Peptide-SA1 (e), Peptide-SA2 (f), Peptide-SE (g) or Peptide-SP (h) at 100µM.** In bold, epimerized residues.

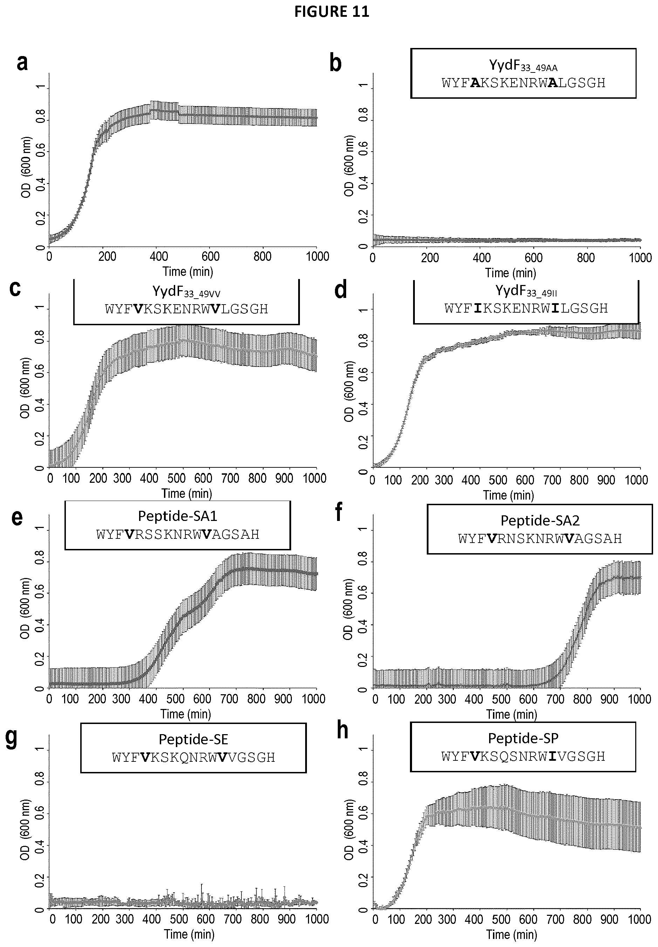

**Figure 11****- Growth of *Enterococcus faecalis* in liquid BHI medium in the presence of YydF₃₃₋₄₉ (a), YydF_{33_49AA} (b), YydF_{33_49VV} (c), YydF_{33_49II} (d), Peptide-SA1 (e), Peptide-SA2 (f), Peptide-SE (g) or Peptide-SP (h)** at **100µM.** In bold, epimerized residues.

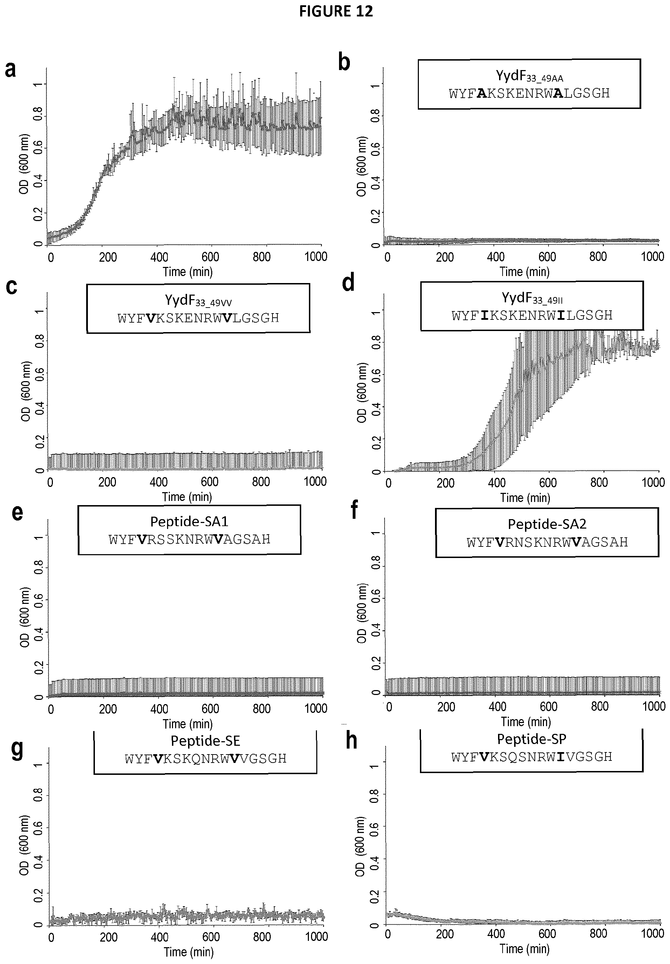

**Figure 12** - **Growth of *Streptococcus agalactiae* in liquid BHI medium in the presence of YydF₃₃₋₄₉ (a), YydF_{33_49AA} (b), YydF_{33_49VV} (c), YydF_{33_49II} (d), Peptide-SA1 (e), Peptide-SA2 (f), Peptide-SE (g) or Peptide-SP (h) at 100µM.** In bold, epimerized residues.

### EXAMPLES

### Example 1

Here the inventors showed that the common laboratory strain *Bacillus subtilis* is able to produce a novel type of bioactive peptides containing D-amino acids despite being of ribosomal origin. This peptide is post-translationally modified by a novel enzyme belonging to the superfamily of radical SAM enzymes. They demonstrated that this novel enzyme uses an unprecedented radical-based mechanism to convert L-Isoleucine and L-valine residues into D-allo-Isoleucine and D-valine. They established that this enzyme generates a 5'-deoxyadenosyl radical to catalyze C_{α} H-atom abstraction leading to the formation of a carbon-centered radical. Mutagenesis experiments support that this enzyme possesses two essential [4Fe-4S] centers and allow identifying a critical H-atom donor, required for the termination of the catalytic cycle. Finally, in a unique manner, they discovered that the presence of D-amino acids is required for the activity of this bioactive peptide which likely induces LiaRS, a major component of the bacterial cell wall integrity.

The Lia system of *Bacillus subtilis* is a cell envelope stress module composed of a two-component system (LiaRS) and an inhibitory protein (LiaF). This genetic system is highly conserved among Firmicutes and part of the complex regulatory network orchestrating the cell wall stress response. Although its regulation has been described in great details, its precise physiological role in *B. subtilis* is not fully understood. LiaRS is specifically and strongly induced by antibiotics targeting the cell wall such as nisin, vancomycin or bacitracin and has thus been developed as a biosensor and high-throughput screen for cell wall antibiotics. Upon antibiotics sensing, LiaRS transduces cell envelop stress signals activating gene expression presumably to maintain cell wall integrity although it does not confer antibiotic resistance.

In an attempt to identify genes involved in LiaRS regulation, a mutagenesis study was undertaken in *B. subtilis* and led to the discovery of the yydFGHIJ operon (Butcher et al, 2007, J Bacteriol, 189, 8616). This operon shows positive regulation on LiaRS and possesses all the characteristic features of a genetic system encoding a putative peptide (YydF) modified by a radical SAM enzyme and a protease (YydG and YydH respectively) then finally exported in the extracellular medium by an ABC-type transporter (YydlJ) even though none of these components were ever isolated or investigated (**Fig. 1A**).

Radical SAM enzymes are an emerging family of enzymes catalyzing a large diversity of protein and peptide modifications such as oxidation, unusual methyl transfer reaction or thioether bond formation. They have emerged as major players for the biosynthesis of the so called RiPPs (Ribosomally synthesized and post-translationally modified peptides) being involved in chemically challenging reactions, that no other enzymes are able to perform. To investigate the biological role and the catalytic function of the putative radical SAM enzyme YydG, the inventors over-expressed the protein in *E. coli* and assayed its activity against the YydF peptide. The purified protein (**Fig. 1B**) had the distinctive spectroscopic properties of radical SAM enzyme with charge transfer absorption band at 320 and 420 nm (**Fig. 1C**). Based on a [4Fe-4S]²⁺ cluster molar extinction coefficient of ε₄₁₀ ~15,000 M⁻¹ cm⁻¹, the enzyme appears to possess one to two [4Fe-4S] centers after anaerobic reconstitution (**Fig. 1C**).

Genome mining revealed that yydF and yydG are also present in several Gram-positive pathogens such as *Enterococcus faecalis* and several *Streptococci* and *Staphylococci* including *S*. *agalactiae* and *S*. *epidermidis.* Sequence alignment of the YydF homologs indicated a putative leader-sequence located in the N-terminus part and a highly conserved motif from the residue 17 to the end of the peptide (**Fig. 1D**). The inventors assayed the reconstituted enzyme either with the full-length YydF peptide or a truncated form, encompassing the conserved amino acid residues from position 18 to 49 that we called YydF₁₈₋₄₉. As shown (**Fig. 1E**), under anaerobic and reducing conditions, YydG produced the expected 5'-deoxyadenosine (5'-dA; eluting at 12.3 min) resulting from the *S-*adenosyl-L-methionine (SAM) homolytic cleavage and also three peptide derivatives: YydFₐ, YydF_{b} & YydF_{c} eluting at 46, 47.6 & 48 min (**Fig. 1E****, F**, **G and H**). The formation of the three peptides was strictly dependent of the presence of sodium dithionite as one-electron donor and similar products were obtained using YydF or **YydF₁₈₋₄₉** (**Fig. 1F,** **G** **and** **H**). Kinetic analysis of the reaction showed that YydG produced one mole of 5'-dA per mole of modified product and catalyzed several turnovers under *in vitro* conditions, although uncoupled SAM cleavage occurred as the reaction proceed (**Fig. 1G**). These results demonstrated that, *in vitro,* YydG used SAM to modify YydF through a radical-based mechanism. Since YydF₁₈₋₄₉ proved to be a better substrate and was easier to characterize, we decided to use it to identify the modification catalyzed by YydG.

Mass spectrometry inspection of the three peptides formed revealed no mass difference compared with the substrate (YydF₁₈₋₄₉ [M+3H]³⁺= 1258.92). This was in contrast with all known rSAM enzymes catalyzing peptide or protein post-translational modifications such as such as cross-linking, oxidation or methylation (*7*, *9*, *14-16*). Tryptic peptide mapping of the substrate gave three peptides (**Peptide 1** [M+H]⁺ =931.48, **Peptide 2** [M+H]²⁺ =592.31 and **Peptide** 3: [M+H]⁺= 768.41) eluting at 22, 27 and 19.2 min) as shown on **Figure 2A****.** Comparison with the enzymatically modified peptide showed the appearance of two new peptides (*i*.*e*. **Peptide 2*** and **Peptide 3***) having the same molecular weight as **Peptide 2** & **3** but eluting at 26.5 and 23.5 min, respectively (**Fig 2B**). This result supported that YydG had introduced two modifications one located internally (in **Peptide 2**) and one in the C-terminus end of peptide (in **Peptide 3**). In all the experiments performed, **YydF_{c}** was the main product. Tryptic peptide mapping revealed that essential the C-terminus end of the peptide was modified since the **Peptide 3^{∗}/Peptide 3** ratio was 5 times larger than the **Peptide 2*/Peptide 2** ratio (**Fig. 2B**) indicating that YydG had some preferred sites.

To identify the nature and location of the modifications catalyzed by YydG, the inventors repeated the reaction in >90% deuterated buffer since rSAM enzymes are known to abstract and sometimes exchange, H-atoms during catalysis. In deuterated buffer, YydG produced a similar product pattern with YydFc being always the most abundant product (**Fig. 2C**). Interestingly, LC-MS analysis of the reaction showed that under these conditions, YydFₐ and YydF_{c} had a molecular weight of [M+3H]³⁺= 1259.24 and YydF_{b} a molecular weight of [M+3H]³⁺= 1259.6. This corresponded to one and two Dalton units more than the substrate, **YydF₁₈₋₄₉** ([M+3H]³⁺= 1258.92), unambiguously demonstrating that one deuterium atom was incorporated into YydFₐ, YydF_{c} and two deuterium atoms in YydF_{b}, respectively. Tryptic peptide mapping of the reaction allowed to localize deuterium incorporation exclusively in the **Peptide 2*** and **Peptide 3*** whose molecular masses shifted from one Dalton unit (i.e. [M+2H]²⁺ =593.04 and [M+2H]⁺= 769.52) **(****Fig. 2D****).** LC-MS/MS fragmentation of these two peptides demonstrated deuterium incorporation on **Val₃₆** in **Peptide 2^{∗}** (as shown by the characteristic ions y₁, y₂+1 and b₇, b₈+1) and on **Ile₄₄** in **Peptide 3^{∗}** (as shown by identification of the ions y₅ and y₆+1). Altogether these results demonstrated that YydG catalyzes the replacement of two peptide H-atoms by two solvent exchangeable H-atoms.

To determine the nature of the modification, the inventors performed acid hydrolysis of the peptide and analyzed its amino acid content, after derivatization with *N*-α-(2,4-dinitro-5-fluorophenyl)-L-valinamide (L-FDVA), by LC-MS. The YydF₁₈₋₄₉ peptide contains two Val and one Ile residues but five Leu residues which not only have the same molecular weight than Ile but also eluted at similar retention times. Optimized LC-MS/MS conditions allowed, as shown on **Figure 3A**, the separation and characterization of L-Ile and L-Leu but also of their D-configured counterparts (D-allo-Ile and D-Leu). Analysis of the enzymatically modified peptides clearly showed that, in addition of the identification of L-Ile and L-Leu, another product eluting at 27.7 min was formed corresponding to D-allo-Ile. Similarly, the analyses of Val residues (**Fig. 3B**) showed the presence not only of L-Val but also of D-Val eluting at 26 min.

The inventors hence established that YydG is a radical SAM epimerase, the first one shown to be active *in vitro* on a peptide backbone. The enzyme catalyzed the epimerization of up to ~35% of the Ile and ~10% of Val residues. Consistent with this conclusion, when one derivatized the amino acids epimerized by incubating YydG in deuterated buffer, their mass analyses revealed a +1Da increment (**Fig. 3C&D**), consistent with the analyses performed on the intact peptide and their tryptic derivatives (**Fig. 2C&D**).

To definitely assert their identity as D-configured amino acids, the inventors synthesized a YydF₁₈₋₄₉ variant peptide containing one D-Val and one D-allo-Ile in positions 36 and 44, respectively. The tryptic peptide mapping and the amino acid profile of this synthetic peptide perfectly reproduced the ones of the enzymatically modified peptide (data not shown).

Based on these analyses, the inventors were able to assign YydFₐ as a peptide containing a D-Val in position 36, YydF_{c} as peptide containing a D-allo-Ile in position 44 and YydF_{b} as peptide containing a D-Val and a D-allo-Ile in positions in positions 36 & 44, respectively. Hence YydG, produced a mixture of peptides containing either a single or double modified amino acids, with Ile₄₄ being the favored substrate (**Fig. 2C**).

During the epimerization reaction performed in deuterium buffer, if the inventors have established that a solvent-exchangeable H-atom is incorporated into in the peptide backbone, the 5'-dA produced contained no significant labeling as shown by LC-M analysis. These results and the kinetic analysis on **Figure 1G** are consistent with YydG producing one 5'-dA radical (5'-dA●) to abstract the C_{α} H-atom of Val₃₆ or Ile₄₄ with the concomitant formation of one mole of 5'-dA.

The last questions which remained to be solved, was the origin of exchangeable H-atom introduced during catalysis. Indeed, the carbon-centered radical was unlikely to interact with a buffer component as such highly reactive species must be kept sealed in the enzyme active site. The inventors favored a protein amino acid residue as H-atom donor and radical quencher required to terminate the reaction. Close inspection of the YydG sequence pointed out that, in addition to the three cysteine residues from the radical SAM motif, only six cysteines were present in the sequence (**Fig. 4A**). Interestingly, two cysteine residues (*i*.*e*. **Cys22** and **Cys223**) were adjacent to another cysteine residue, one of which being inside the predicted loop containing the rSAM [4Fe-4S] center. The organization of the five other cysteine residues in the C-terminus end of the protein was reminiscent of motifs involved in the coordination of additional [4Fe-4S] centers in rSAM enzymes. To probe their function, the inventors substituted the three cysteine residues of the CxxxCxxC radical SAM motif, Cys22, Cys222 or Cys223 by alanine residues. The four designed mutants (*i*.*e*. **A3**, **C22A**, **C222A** and **C223A**) were successfully purified although the **C222A** mutant proved to be recalcitrant to purification and produced partly as a truncated form (**Fig. 4B**). Spectroscopic analysis showed that, based on its UV-visible spectrum, the AxxxAxxA mutant contained ~1 [4Fe-4S] center while the amount of [4Fe-4S] center was two-times higher in the C22A and the C223A mutants (**Fig. 4C**). Importantly, the aerobically purified AxxxAxxA mutant already contained high amounts of iron-sulfur center demonstrating that the presence of [4Fe-4S] center in this mutant was independent of the anaerobic reconstitution. The UV-visible spectra of C22A and C223A mutants perfectly superimpose with the wild-type enzyme (**Fig. 1B**), supporting the fact that YydG likely contains two [4Fe-4S] centers. The C222A mutant appeared to contain no iron-sulfur center, even after anaerobic reconstitution (**Fig. 4C**). The C222A absorption maximum was shifted toward 250 nm indicating that the protein was likely miss-folded, as it has been repeatedly reported when cysteine residues involved in [4Fe-4S] coordination are mutated in iron-suflur enzymes including rSAM enzymes. It is thus likely than Cys222, is involved in coordination of the second [4Fe-4S] center present in YydG.

The activity of all the mutants was assayed with the YydF₁₈₋₄₉ substrate (**Fig. 4D**). As expected, the A3 mutant was unable to convert the peptide substrate and to cleave SAM. The **C222A** mutant was also totally impaired for enzyme activity. In contrasts, the **C22A** mutation did not affect the activity of the enzyme and the three epimerized peptides were produced (*i*.*e*. YydFₐ, YydF_{b} and YydF_{c}). The **C223A** mutation did not either prevent the epimerization activity. However, this enzyme variant produced other peptide derivatives eluting at 10 min, 19.8min and 26 min (**Fig. 4E**). High-resolution mass spectrometry showed these peptides to have a mass shift of -30.005 Da or -1.032 Da, compared to predicted hydrolytic products. They all contained at their C-terminus or N-terminus ends a truncated Val₃₆ or Ile₄₄, the targets of the YydG enzyme (**Fig. 4E**). Their structure was determined as: Ac-GLLDESQKLAKVNDLWYFVKSKENRWI* (SEQ ID No 53) (peptide G₁₈-I₄₄^{∗}, [M+2H]²⁺= 1646.3834) ; and **I**^{∗}LGSGH-NH₂ (SEQ ID No 55) (peptide G₁₈-I₄₄^{∗}, [M+Na]⁺= 603.2851). The truncation was identified as the loss of the amino acid carboxylic or amino group, resulting from the rupture of either the C_{α}-N or the C_{α}-CO bonds, and the addition of an oxygen-atom on the amino acid C_{α}-atom. These results are reminiscent of the substrate fragmentation obtained with another member of the rSAM enzyme family, the pyruvate formate lyase activase, when the reaction was exposed to molecular oxygen. They also definitively established that YydG generates a carbon-centered radical on the C_{α}-atom of Val₃₆ and Ile₄₄ and that Cys223 plays a critical role for the termination of the reaction.

In light of the previous work of the inventors on another rSAM enzyme, the spore photoproduct lyase (SP lyase), the inventors interpreted the role of Cys223 as the critical H-atom donor. Indeed, while investigating a mutant of the SP lyase, they have shown that in the absence of a suitable protein H-atom donor, the substrate radical intermediate can react with adventitious radical scavengers leading to the formation of various adducts. Here, the stabilized C_{α} radical, in the absence of the thiol group of Cys223, is free to react with molecular oxygen leading to these unique peptidyl backbone breakages.

Finally, since the YydFGHIJ operon (**Fig. 1A**) was shown to activate the Lia system in *B. subtilis,* the inventors assayed the activity of the YydF₁₈₋₄₉ peptide before and after enzyme reaction on various bacterial strains. They evidenced strong inhibition growth of *β. subtilis* with either the enzymatically epimerized peptide or the synthetic peptide containing D-Val₃₆ and D-allo-Ile₄₄. In contrast, the unmodified YydF₁₈₋₄₉ peptide was devoid of activity (**Fig. 5A**). Similarly, in liquid medium, only the epimerized peptide proved to be active as a strong and persistent inhibition could be measured (**Fig 5B**). Further studies will be required to decipher the molecular basis of this inhibition but according to the inventors' knowledge, it is the first time that a naturally epimerized peptide proved to be the active form of a regulatory or antimicrobial bacterial peptide.

The present study demonstrates that peptides containing D-amino acids, called herein Epipeptides, are much more common than previously anticipated in living organisms including the common laboratory bacterium *Bacillus subtilis* but also many pathogenic species such as *Streptococcus agalactiae, Enterococcus faecails* or *Staphylococcus epidermidis.* Unexpectedly, the inventors demonstrated here that D-amino acids appear not only to provide resistance to proteases but are directly involved in bacterial response.

### Example 2

A gene, *yydF*, was proposed in the literature to encode a peptide produced by *Bacillus subtilis* (SEQ ID No 1). However no proof of its actual synthesis or of any post-translational modification has been reported.

After growth of *B. subtilis* in a synthetic medium (Buffer solution 5X (Na2HPO4: 17g ; KH2PO4: 7.5g, NaCl: 1.25g; NH4Cl : 2.5g in 500 mL) ; Trace element solution : MnCl₂: 20mg; ZnCl₂: 34mg; CuCl₂: 8.6mg; CoCl₂: 12mg; Na₂MoO₄ :12mg; in 200 mL), the inventors successfully purified a peptide, originating from YydF and encompassing residues 33 to 49 (Figure 7), as established by mass spectrometry analysis (Table 1). The sequence of the isolated peptide was determined to be (**SEQ ID No 61**):

Trp Tyr Phe Val Lys Ser Lys Glu Asn Arg Trp Ile Leu Gly Ser Gly His
1               5                   10                  15
SEQ ID No 61: Sequence of the peptide YydF33_49

In addition, the inventors determined that the peptide YydF₃₃₋₄₉, produced by *B. subtilis* contained 2 epimerized residues (*i*.*e*. D-amino acids) located in position 36 (Val) and 44 (Ile). The peptide was thus called YydF__{33-49DD}. Previous work from the inventors (Example 1) has established that the epimerized residues are the result of the conversion of L-amino acid residues by a unique radical SAM enzyme, YydG, which targets the amino acids C_{α}-atom. Currently, no such short peptides, containing discreet epimerization, are known to be produced by bacteria.

Because the operon YydFGHIJ, was shown to induce the two component system LiaRS, which among other stimuli, sense the bacterial cell-wall integrity, the inventors searched for a putative bacterial growth inhibition triggered by various YydF peptide derivatives. Initial tests were performed with a peptide encompassing residues 18-49 (YydF₁₈₋₄₉, **SEQ ID No 20**).

As shown (**Figure 5B**), only in the presence of the peptide YydF_{18-49DD} containing two epimerized residues: Val₃₆ and Ile₄₄ (numbering according to SEQ ID No 61), the inventors monitored bacterial growth inhibition. The presence of one epimerized residues or the absence of epimerized residues did not significantly impacted bacterial growth. This demonstrated, for the first time, that a short peptide with epimerized residues can inhibit bacterial growth.

Having established that the presence of two key epimerized residues is critical for the inhibitory properties, the inventors synthesized two peptides corresponding to the sequence of the peptide produced by *B. subtilis* (**SEQ ID No 61**). These two peptides contained either only L-amino acid residues (YydF₃₃₋₄₉) or the two critical epimerized residues: D-Val₃₆ and D-Ile₄₄ (YydF_{33-49DD}). Only the YydF_{33-49DD} peptide proved to inhibit bacterial growth (**Figure 8a**). This novel peptide proved to be 100 times more potent than the YydF_{18-49DD} peptide previously assayed (**Figure 8b****&c**) with a MIC <1µM. In addition, the inventors showed that these peptides do not only inhibit bacterial growth but they also induce bacterial cell death. Indeed, if after initial growth for 3 hours, the YydF₁₈₋₄₉ peptide is added at mid-exponential phase (**Figure 9**), a clear slow down followed by a decrease of the cell density was measured.

Interestingly, homologs of the YydF peptides are predicted in the genome of several Gram-positive bacteria such as: *Salinibacillus aidingensis, Bacillus coagulans, Paenibacillus sp* and several pathogenic species such as: *Enterococcus faecalis, Enterococcus caccae, Streptococcus agalactiae, Staphylococcus pseudintermedius, Staphylococcus equorum, Staphylococcus condimenti* and *Staphylococcus epidermidis* (**Figure 1D****,** **Figure 6**).

In order to determine if these peptides are bioactive, the inventors synthesized a library of peptides based on the sequences identified in the genomes of *Streptococcus* and *Staphylococcus* species. They hypothesized that these peptides should contain the same post-translational modifications as the ones identified in *B. subtilis,* which means a processed peptide of 17 amino acid residues with two D-amino acids in the positions 4 and 12 (**SEQ ID Nos 62-65**). The epimerized residues are in bold.

| | |
|---|---|
| Streptococcus agalactiae Peptide-SA1 | WYF**V**RSSKNRW**V**AGSAH (SEQ ID No 62) |
| Streptococcus agalactiae Peptide-SA2 | WYF**V**RNSKNRW**V**AGSAH (SEQ ID No 63) |
| Staphylococcus equorum Peptide-SE | WYF**V**KSKQNRW**V**VGSGH (SEQ ID No 64) |
| Staphylococcus pseudintermedius Peptide-SP | WYF**V**KSQSNRW**I**VGSGH (SEQ ID No 65) |

In addition, the inventors also synthesized three unnatural peptides derived from the *B. subtilis* YydF_{33_49} sequence (**SEQ ID No 61**) but for which the two epimerized residues (*i*.*e*. Val₃₆ and Ile₄₄) were both substituted by Val, Ile or Ala residues, YydF_{33_49VV}, YydF_{33-49II} and YydF_{33_49AA},respectively (SEQ ID Nos 66-68). The epimerized residues are in bold.

| | |
|---|---|
| YydF_{33_49AA} | WYF**A**KSKENRW**A**LGSGH (SEQ ID No 66) |
| YydF_{33_49VV} | WYF**V**KSKENRW**V**LGSGH (SEQ ID No 67) |
| YydF_{33_49II} | WYF**I**KSKENRW**I**LGSGH (SEQ ID No 68) |

These 7 peptides (**SEQ ID Nos 62-68**) were assayed against *B*. *subtilis* and the two representative Gram-positive pathogens: *S*. *agalactiae* and *E. faecalis.* As shown, all peptides were effective against *B. subtilis* including the peptides with unnatural sequences (**Figure 10**). *E. faecalis* growth was significantly delayed with Peptide-SA1 & Peptide-SA2 and growth totally inhibited with Peptide-SE and the unnatural peptide YydF_{33_49AA} (**Figure 11**). *S*. *agalactiae* was inhibited by YydF_{33-49DD} and the derivatives YydF_{33-49AA} and YydF_{33-49VV} but not by YydF_{33-49II} (**Figure 12**). The peptides: Peptide-SA1, Peptide-SA2, Peptide-SE and Peptide-SP were all inhibitors.

The inventors thus demonstrated that short peptides containing two D-amino acid residues are a novel class of inhibitory peptides able to inhibit the growth of several Gram-positive bacteria including relevant pathogens. They are efficient whether added at the beginning or after bacterial growth at mid-exponential phase. Finally, some discreet modifications in the sequence are able to tune the inhibition properties and the specificities at the genera and species level of these peptides allowing the development of targeted antibiotics. In addition, based on the framework of 17 amino acids and the conserved location of two D-amino acids (in position 4 and 12) downstream to aromatic residues (W or Y), the inventors also demonstrated that it is possible to design peptides with unnatural sequences that proved to be effective against all the Gram-positive bacteria assayed.

The bioactive peptides proved to have sequence identity varying from 100 to 58.8% relative to the original YydF₃₃₋₄₉ sequence which means at least 7 amino acid residues could be changed without altering their global inhibition properties. It is thus possible to engineer these peptides in an unprecedented manner to target specific bacterial genera and tune their biological properties.

| # | Percent | Identity | Matrix # | | | | | | |
|---|---|---|---|---|---|---|---|---|---|
| 1: | Peptide-SA1 | 100.00 | 94.12 | 64.71 | 70.59 | 58.82 | 58.82 | 64.71 | 70.59 |
| 2: | Peptide-SA2 | 94.12 | 100.00 | 58.82 | 64.71 | 52.94 | 52.94 | 58.82 | 64.71 |
| 3: | Peptide-SP | 64.71 | 58.82 | 100.00 | 82.35 | 70.59 | 76.47 | 82.35 | 76.47 |
| 4: | Peptide-SE | 70.59 | 64.71 | 82.35 | 100.00 | 76.47 | 76.47 | 82.35 | 88.24 |
| 5: | YydF33_49AA | 58.82 | 52.94 | 70.59 | 76.47 | 100.00 | 88.24 | 88.24 | 88.24 |
| 6: | YydF33_49II | 58.82 | 52.94 | 76.47 | 76.47 | 88.24 | 100.00 | 94.12 | 88.24 |
| 7: | YydF33-49DD | 64.71 | 58.82 | 82.35 | 82.35 | 88.24 | 94.12 | 100.00 | 94.12 |
| 8: | YydF33_49VV | 70.59 | 64.71 | 76.47 | 88.24 | 88.24 | 88.24 | 94.12 | 100.00 |

### Materials and Methods

### YydG expression

The *yydG* genes was synthesized (Life Technologies) and cloned into a pASK plasmid. The plasmid was expressed in *E. coli* BL21 (DE3) star (Life Technologies) and protein expression was performed in LB medium containing ampicillin (100 µg.mL-1). After overnight growth at 21°C, the cells were collected and disrupted by ultra-sonication in buffer A (Tris 50 mM, KCI 300 mM, Glycerol 10% pH 7.5). The bacterial suspension was centrifuged at 45,000 x g for 1.5 hours and the protein supernatant was loaded onto a Streptactin high capacity (IBA GmbH) column previously equilibrated with buffer A. The YydG protein was eluted with 6mL of buffer A containing desthiobiotine (0.6 mg/mL) further concentrated with Amicon concentrator (Millipore) with a molecular cut-off of 10 kDa.

### Enzyme reconstitution

YydG was reconstituted under anaerobic conditions in a Bactron IV anaerobic chamber. The protein was mixed with 3 mM of DTT at 12°C during 15 minutes then Na₂S and (NH₄)₂Fe(SO₄)₂ were added and the solution was incubated at 12°C during 4h.

### Enzyme assays

YydG was incubated with 3 mM DTT, 1 mM SAM and 1 mM peptide substrate unless otherwise indicated. Incubations were performed at 25°C under strict anaerobic conditions and 10µL aliquots sampled overtime.

### HPLC analysis

HPLC analysis was performed on an Agilent 1200 series infinity equipped with a reversed phase column (LiChroSphere 100 RP-18e 5 µm) (Merck Millipore). A gradient from solvent A (H₂O, 0.1% TFA) to B (80 % CH₃CN, 19.9 % H₂O, 0.1% TFA) was applied as follow: 0-1 min: 100% A/0% B; 1-45 min : a linear gradient with 1% of solvent B per minute at a flow rate of 1 ml.min⁻¹. Detection was made at 257 & 278 nm with a diode array detector and by fluorescence (excitation at 278 nm and emission at 350 nm).

### Liquid chromatography-mass spectrometry /mass spectrometry analysis

High resolution liquid chromatography-mass spectrometry /mass spectrometry analysis were performed using an LTQ-Orbitrap Discovery mass spectrometer (ThermoFisher) with a nanoelectrospray ion source and an Ultimate 3000 LC system (Dionex). A LTQ mass spectrometer (ThermoFisher) with a nanoelectrospray ion source was used for routine analysis. Peptide analysis was performed on a nanocolumn Pepmap 100 C18 (0.075 by 15 cm, 100 Å, 3 µm).

### Inhibition assay on solid medium

An overnight culture of the bacterial strain to be assayed was freshly inoculated to sterile BHI liquid medium. After 4 hours of bacterial growth at 37°C, the medium was diluted to 1/1000 and inoculated into a soft agarose medium pre-heated at 42°C. The agarose medium containing bacteria was overlaid on a previously jellified sterile BHI agarose layer. 200 µg of peptide was spotted onto the plate and bacterial growth proceeded at 37°C.

### Inhibition assay on liquid medium

An overnight culture of the bacterial strain to be assayed was freshly inoculated to sterile LB liquid medium. After 4 hours of bacterial growth at 37°C, the medium was diluted to 1/10,000 and inoculated into sterile liquid LB or BHI medium. Peptide solution was added (1/100) to a final concentration ranging from 0.01 to 100µM and OD at 600 nm was recorded continuously using a Tecan microplate reader (Infinite^{®} 200 PRO series).

**Table 1**

| **Mass fragments for peptide YydF₃₃₋₄₉ isolated from *B. subtilis*** | | | | | | |
|---|---|---|---|---|---|---|
| Sequence | | b+ | b++ | y+ | y++ | |
| W | 1 | 187.08718 | 94.04753 | 2107.08779 | 1054.04783 | 17 |
| Y | 2 | 350.15051 | 175.57919 | 1921.00848 | 961.00818 | 16 |
| F | 3 | 497.21892 | 249.11340 | 1757.94515 | 879.47651 | 15 |
| V | 4 | 596.28734 | 298.64760 | 1610.87674 | 805.94230 | 14 |
| K | 5 | 724.38230 | 362.69509 | 1511.80833 | 756.40810 | 13 |
| S | 6 | 811.41433 | 406.21110 | 1383.71336 | 692.36062 | 12 |
| K | 7 | 939.50929 | 470.25858 | 1296.68133 | 648.84460 | 11 |
| E | 8 | 1068.55188 | 534.77988 | 1168.58637 | 584.79712 | 10 |
| N | 9 | 1182.59481 | 591.80134 | 1039.54378 | 520.27582 | 9 |
| R | 10 | 1338.69592 | 669.85190 | 925.50085 | 463.25436 | 8 |
| W | 11 | 1524.77523 | 762.89155 | 769.39974 | 385.20381 | 7 |
| I | 12 | 1637.85930 | 819.43358 | 583.32043 | 292.16415 | 6 |
| L | 13 | 1750.94336 | 875.97562 | 470.23637 | 235.62212 | 5 |
| G | 14 | 1807.96483 | 904.48635 | 357.15230 | 179.08009 | 4 |
| S | 15 | 1894.99685 | 948.00236 | 300,13084 | 150,56935 | 3 |
| G | 16 | 1952.01832 | 976.51309 | 213.09881 | 107.05334 | 2 |
| H | 17 | 2089.07723 | 1045.04255 | 156.07735 | 78.54261 | 1 |

| | |
|---|---|
| (M) | 2106.07997 |
| (M+H)⁺ | 2107.08779 |
| (M+2H)²⁺ | 1054.04783 |
| (M+3H)³⁺ | **703.03451** |
| (M+4H)⁴⁺ | 527.52785 |

## Claims

1. A peptide having an antibacterial activity having at least two D-configured amino acids, comprising a sequence of 17 residues having at least 40 % of identity with the sequence in positions 33 to 49 of SEQ ID No 1 and said sequence comprising the sequences W-Y-F-[V/I/A] (SEQ ID No 71) and W-[I/V/A]-X4-G-S (SEQ ID No 69), wherein X4 is any amino acid, and a connecting sequence between SEQ ID No 69 and SEQ ID No 71 of 4-8 amino acids, wherein the residues [V/I/A] of SEQ ID Nos 69 and 71 have a D-configuration and wherein the peptide has a length from about 17 to about 35 amino acids.

2. The peptide according to claim 1, wherein the peptide comprises a sequence of W-Y-F-[V/I/A]-[K/R]-Xa-Xb-Xc-N-R-W-[V/I/A]-Xd-G-S-Xe-H (SEQ ID No 72), wherein Xa, Xb and Xc are a polar amino acid, Xd is an aliphatic amino acid and Xe is any amino acid.

3. The peptide according to claim 2, wherein the peptide comprises a sequence of W-Y-F-[V/I/A]-[K/R]-[S/N]-[S/Q/K]-[E/K/S/Q]-N-R-W-[V/I/A]-[L/V/A]-G-S-[A/G]-H (SEQ ID No 73).

4. The peptide according to any one of claims 1-3, wherein the peptide comprises a sequence selected from the group consisting of SEQ ID Nos 61-68.

5. The peptide according to claim 1, wherein the peptide comprises a sequence of 24 residues having at least 40 % of identity with the sequence in positions 26 to 49 of SEQ ID No 1 and said sequence comprising the sequences N/D-D-L-W-Y-F-[V/I/A] (SEQ ID No 21) and W-[I/V/A]-X4-G-S (SEQ ID No 69), wherein X4 is any amino acid, and a connecting sequence between SEQ ID No 21 and SEQ ID No 69 of 4-7 amino acids, and wherein the peptide has a length from about 25 to about 35 amino acids.

6. The peptide according to claim 5, wherein the peptide comprises the sequences L-X1-X2-X3-N-D-L-W-Y-F-V/I (SEQ ID No 23), and W-I/V-X4-G-S (SEQ ID No 22), wherein X1, X2, X3 and X4 are any amino acid.

7. The peptide according to claim 6, wherein X1 is Alanine, Aspartic acid or Glutamic acid, X2 is Lysine, Asparagine or Aspartic acid and X3 is Valine, Isoleucine or Glutamine.

8. The peptide according to claim 5, wherein the peptide comprises the sequences L-A-K-V-N-D-L-W-Y-F-V (SEQ ID No 24), and W-I/V-X4-G-S (SEQ ID No 22), wherein X4 is a hydrophobic amino acid, preferably Leucine, Valine, Alanine or Methionine.

9. The peptide according to any one of claims 5-8, wherein the peptide comprises, consists essentially of or consists of the sequence of SEQ ID No 20 and the Valine in position 19 is in D-configuration and the isoleucine in position 27 is in D-configuration.

10. A pharmaceutical composition comprising a peptide according to any one of claims 1-9.

11. The peptide according to any one of claims 1-9 for use as a drug, preferable as an antimicrobial, more preferably as an antibacterial.

12. An *in vitro* method for preparing a peptide according to any one of claims 1-9, wherein the method comprises a step of contacting a peptide comprising a sequence of 17 residues having at least 40 % of identity with the sequence in positions 33 to 49 of SEQ ID No 1 and said sequence comprising the sequences W-Y-F-[V/I/A] (SEQ ID No 71) and W-[I/V/A]-X4-G-S (SEQ ID No 69), wherein X4 is any amino acid, and a connecting sequence between SEQ ID No 69 and SEQ ID No 71 of 4-8 amino acids and wherein the peptide has a length from about 17 to about 35 amino acids, with an epimerase having an amino acid sequence having at least 30 % of identity with SEQ ID No 25 and in which 70% of the positions 14-15, 18, 20-24, 27, 58-60, 63, 83, 84, 87-90, 112, 115, 117, 120, 150, 152, 169, 176, 180, 181, 183, 204, 206-208, 214, 217, 220-225, 228, 230, 252, 254, 262, 289, 292, 296, and 309 of SEQ ID No 25 are conserved and all the positions 14-15, 18, 20-24, 27, 58-60, 63, 83, 84, 87-90, 112, 115, 117, 120, 204, 206-208, 214, 217, 220-225, 228, and 230 of SEQ ID No 25 are conserved.

13. The *vitro* method of claim 12, wherein the peptide comprising a sequence of 24 residues having at least 40 % of identity with the sequence in positions 26 to 49 of SEQ ID No 1 and said sequence comprising the sequences N/D-D-L-W-Y-F-V/I (SEQ ID No 21) and W-I/V-X4-G-S (SEQ ID No 22), wherein X4 is any amino acid, and a connecting sequence between SEQ ID No 21 and SEQ ID No 22 of 4-7 amino acids, and wherein the peptide has a length from about 25 to about 35 amino acids.

14. Use of a radical SAM peptide epimerase having an amino acid sequence having at least 30 % of identity with SEQ ID No 25 and in which 70% of the positions 14-15, 18, 20-24, 27, 58-60, 63, 83, 84, 87-90, 112, 115, 117, 120, 150, 152, 169, 176, 180, 181, 183, 204, 206-208, 214, 217, 220-225, 228, 230, 252, 254, 262, 289, 292, 296, and 309 of SEQ ID No 25 are conserved and all the positions 14-15, 18, 20-24, 27, 58-60, 63, 83, 84, 87-90, 112, 115, 117, 120, 204, 206-208, 214, 217, 220-225, 228, and 230 of SEQ ID No 25 are conserved for preparing a peptide according to any one of claims 1-9.

15. A recombinant host cell comprising a heterologous nucleic acid encoding a peptide comprising a sequence of 17 residues having at least 40 % of identity with the sequence in positions 33 to 49 of SEQ ID No 1 and comprising the sequences W-Y-F-[V/I/A] (SEQ ID No 71) and W-[I/V/A]-X4-G-S (SEQ ID No 69), wherein X4 is any amino acid, and a connecting sequence between SEQ ID No 69 and SEQ ID No 71 of 4-8 amino acids and wherein the peptide has a length from about 17 to about 35 amino acids, and a heterologous nucleic acid encoding a radical SAM peptide epimerase having an amino acid sequence having at least 30 % of identity with SEQ ID No 25 and in which 70% of the positions 14-15, 18, 20-24, 27, 58-60, 63, 83, 84, 87-90, 112, 115, 117, 120, 150, 152, 169, 176, 180, 181, 183, 204, 206-208, 214, 217, 220-225, 228, 230, 252, 254, 262, 289, 292, 296, and 309 of SEQ ID No 25 are conserved and all the positions 14-15, 18, 20-24, 27, 58-60, 63, 83, 84, 87-90, 112, 115, 117, 120, 204, 206-208, 214, 217, 220-225, 228, and 230 of SEQ ID No 25 are conserved and being able to co-express the peptide and the epimerase.

16. The recombinant host cell of claim 15, wherein the peptide comprises a sequence of 24 residues having at least 40 % of identity with the sequence in positions 26 to 49 of SEQ ID No 1 and comprising the sequences N/D-D-L-W-Y-F-[V/I/A] (SEQ ID No 21) and W-I/V-X4-G-S (SEQ ID No 22), wherein X4 is any amino acid, and wherein the peptide has a length from about 25 to about 35 amino acids.

17. An method for preparing a peptide according to any one of claims 1-9, wherein the method comprises culturing a recombinant host cell according to claim 15 or 16 in conditions suitable for the co-expression of the peptide and the epimerase and recovering the peptide having at least two D-configured amino acid.

18. Use of a recombinant host cell according to any one of claims 15 and 16 for preparing a peptide according to any one of claims 1-9.

## Patentansprüche

1. Peptid mit antibakterieller Aktivität mit mindestens zwei D-konfigurierten Aminosäuren, umfassend eine Sequenz von 17 Resten mit mindestens 40 % Identität mit der Sequenz in den Positionen 33 bis 49 von SEQ ID Nr. 1, wobei die Sequenz die Sequenzen W-Y-F-[V/I/A] (SEQ ID Nr. 71) und W-[I/V/A]-X4-G-S (SEQ ID Nr. 69) umfasst, wobei X4 eine beliebige Aminosäure ist, und eine Verbindungssequenz von 4-8 Aminosäuren zwischen SEQ ID Nr. 69 und SEQ ID Nr. 71, wobei die Reste [V/1/A] von SEQ ID Nr. 69 und 71 eine D-Konfiguration haben und wobei das Peptid eine Länge von etwa 17 bis etwa 35 Aminosäuren hat.

2. Peptid nach Anspruch 1, wobei das Peptid eine Sequenz von W-Y-F-[V/I/A]-[K/R]-Xa-Xb-Xc-N-R-W-[V/I/A]-Xd-G-S-Xe-H (SEQ ID Nr. 72) umfasst, wobei Xa, Xb und Xe eine polare Aminosäure sind, Xd eine aliphatische Aminosäure ist und Xe eine beliebige Aminosäure ist.

3. Peptid nach Anspruch 2, wobei das Peptid eine Sequenz von W-Y-F- [V/I/A]-[K/R]-[S/N]-[S/Q/K]-[E/K/S/Q]-N-R-W-[V/I/A]-[L/V/A]-G-S-[A/G]-H (SEQ ID Nr 73) umfasst.

4. Peptid nach einem der Ansprüche 1-3, wobei das Peptid eine Sequenz umfasst, ausgewählt aus der Gruppe, bestehend aus SEQ ID Nr. 61-68.

5. Peptid nach Anspruch 1, wobei das Peptid eine Sequenz von 24 Resten mit mindestens 40 % Identität mit der Sequenz in den Positionen 26 bis 49 von SEQ ID Nr. 1 umfasst und diese Sequenz die Sequenzen N/D-D-L-W-Y-F-[V/I/A] (SEQ ID Nr. 21) und W-[I/V/A]-X4-G-S (SEQ ID Nr. 69) umfasst, wobei X4 eine beliebige Aminosäure ist, und eine Verbindungssequenz zwischen SEQ ID Nr. 21 und SEQ ID Nr. 69 aus 4-7 Aminosäuren und wobei das Peptid eine Länge von etwa 25 bis etwa 35 Aminosäuren hat.

6. Peptid nach Anspruch 5, wobei das Peptid die Sequenzen L-X1-X2-X3-N-D-L-W-Y-F-V/I (SEQ ID Nr. 23) und W-I/V-X4-G-S (SEQ ID Nr. 22) umfasst, wobei X1, X2, X3 und X4 beliebige Aminosäuren sind.

7. Peptid nach Anspruch 6, wobei X1 Alanin, Asparaginsäure oder Glutaminsäure ist, X2 Lysin, Asparagin oder Asparaginsäure ist und X3 Valin, Isoleucin oder Glutamin ist.

8. Peptid nach Anspruch 5, wobei das Peptid die Sequenzen L-A-K-V-N-D-L-W-Y-F-V (SEQ ID Nr. 24) und W-I/V-X4-G-S (SEQ ID Nr. 22) umfasst, wobei X4 eine hydrophobe Aminosäure ist, vorzugsweise Leucin, Valin, Alanin oder Methionin.

9. Peptid nach einem der Ansprüche 5-8, wobei das Peptid die Sequenz von SEQ ID Nr. 20 umfasst, im Wesentlichen daraus besteht oder daraus besteht und das Valin in Position 19 in D-Konfiguration ist und das Isoleucin in Position 27 in D-Konfiguration ist.

10. Pharmazeutische Zusammensetzung, umfassend ein Peptid nach einem der Ansprüche 1-9.

11. Peptid nach einem der Ansprüche 1-9 zur Verwendung als Arzneimittel, vorzugsweise als antimikrobielles Mittel, stärker bevorzugt als antibakterielles Mittel.

12. In-vitro-Verfahren zur Herstellung eines Peptids nach einem der Ansprüche 1 bis 9, wobei das Verfahren einen Schritt des Inkontaktbringens eines Peptids umfasst, das eine Sequenz von 17 Resten mit mindestens 40% Identität mit der Sequenz in den Positionen 33 bis 49 von SEQ ID Nr. 1 umfasst und wobei diese Sequenz die Sequenzen W-Y-F-[V/I/A] (SEQ ID Nr. 71) und W-[I/V/A]-X4-G-S (SEQ ID Nr. 69) umfasst, wobei X4 eine beliebige Aminosäure ist, und eine Verbindungssequenz von 4-8 Aminosäuren zwischen SEQ ID Nr. 69 und SEQ ID Nr. 71 und wobei das Peptid eine Länge von etwa 17 bis etwa 35 Aminosäuren aufweist, mit einer Epimerase, die eine Aminosäuresequenz mit mindestens 30 % Identität mit SEQ ID Nr. 25 aufweist und in der 70 % der Positionen 14-15, 18, 20-24, 27, 58-60, 63, 83, 84, 87-90, 112, 115, 117, 120, 150, 152, 169, 176, 180, 181, 183, 204, 206-208, 214, 217, 220-225, 228, 230, 252, 254, 262, 289, 292, 296 und 309 von SEQ ID Nr. 25 konserviert sind und alle Positionen 14-15, 18, 20-24, 27, 58-60, 63, 83, 84, 87-90, 112, 115, 117, 120, 204, 206-208, 214, 217, 220-225, 228 und 230 von SEQ ID Nr. 25 konserviert sind.

13. In-vitro-Verfahren nach Anspruch 12, wobei das Peptid eine Sequenz von 24 Resten mit mindestens 40 % Identität mit der Sequenz in den Positionen 26 bis 49 von SEQ ID Nr. 1 umfasst und die Sequenz die Sequenzen N/D-D-L-W-Y-F-V/I ( SEQ ID Nr. 21) und W-I/V-X4-G-S (SEQ ID Nr. 22) umfasst, wobei X4 eine beliebige Aminosäure ist, und eine Verbindungssequenz von 4-7 Aminosäuren zwischen SEQ ID Nr. 21 und SEQ ID Nr. 22, und wobei das Peptid eine Länge von etwa 25 bis etwa 35 Aminosäuren hat.

14. Verwendung einer radikalische SAM-Peptid-Epimerase mit einer Aminosäuresequenz mit mindestens 30 % Identität mit SEQ ID Nr. 25 und in der 70 % der Positionen 14-15, 18, 20-24, 27, 58-60, 63, 83, 84, 87-90, 112,115,117,120,150,152,169,176,180,181,183, 204, 206-208, 214, 217, 220-225, 228, 230, 252, 254, 262, 289, 292, 296 und 309 von SEQ ID Nr. 25 konserviert sind und alle Positionen 14-15, 18, 20-24, 27, 58-60, 63, 83, 84, 87-90, 112, 115, 117, 120, 204, 206-208, 214, 217, 220-225, 228 und 230 von SEQ ID Nr. 25 konserviert sind, um ein Peptid nach einem der Ansprüche 1-9 herzustellen.

15. Rekombinante Wirtszelle, umfassend eine heterologe Nukleinsäure, die ein Peptid kodiert, umfassend eine Sequenz von 17 Resten mit mindestens 40 % Identität mit der Sequenz in den Positionen 33 bis 49 von SEQ ID Nr. 1 und umfassend die Sequenzen W-Y-F-[V/I/A] (SEQ ID Nr. 71) und W-[I/V/A]-X4-G-S (SEQ ID Nr. 69), wobei X4 eine beliebige Aminosäure ist, und umfassend eine Verbindungssequenz von 4-8 Aminosäuren zwischen SEQ ID Nr. 69 und SEQ ID Nr 71, wobei das Peptid eine Länge von etwa 17 bis etwa 35 Aminosäuren aufweist, und eine heterologe Nukleinsäure, die eine radikalische SAM-Peptid-Epimerase mit einer Aminosäuresequenz mit mindestens 30 % Identität mit SEQ ID Nr 25 aufweist, und in der 70% der Positionen 14-15, 18, 20-24, 27, 58-60, 63, 83, 84, 87-90, 112, 115, 117, 120, 150, 152, 169, 176, 180, 181, 183, 204, 206-208, 214, 217, 220-225, 228, 230, 252, 254, 262, 289, 292, 296 und 309 von SEQ ID Nr. 25 konserviert sind und alle Positionen 14-15, 18, 20-24, 27, 58-60, 63, 83, 84, 87-90, 112, 115, 117, 120, 204, 206-208, 214, 217, 220-225, 228 und 230 von SEQ ID Nr. 25 sind konserviert sind, und die das Peptid und die Epimerase co-exprimieren kann.

16. Rekombinante Wirtszelle nach Anspruch 15, wobei das Peptid eine Sequenz von 24 Resten mit mindestens 40 % Identität mit der Sequenz in den Positionen 26 bis 49 von SEQ ID Nr. 1 umfasst und die Sequenzen N/D-D-L-W-Y-F-[V/I/A] (SEQ ID Nr. 21) und W-I/V-X4-G-S (SEQ ID Nr. 22), wobei X4 eine beliebige Aminosäure ist und wobei das Peptid eine Länge von etwa 25 bis etwa 35 Aminosäuren hat.

17. Verfahren zur Herstellung eines Peptids nach einem der Ansprüche 1-9, wobei das Verfahren das Kultivieren einer rekombinanten Wirtszelle nach Anspruch 15 oder 16 unter Bedingungen umfasst, die für die Co-Expression des Peptids und der Epimerase und das Gewinnen des Peptids mit mindestens zwei D-konfigurierten Aminosäuren geeignet sind.

18. Verwendung einer rekombinanten Wirtszelle nach einem der Ansprüche 15 und 16 zur Herstellung eines Peptids nach einem der Ansprüche 1-9.

## Revendications

1. Peptide ayant une activité antibactérienne ayant au moins deux acides aminés en configuration D, comprenant une séquence de 17 résidus ayant une identité d'au moins 40 % avec la séquence aux positions 33 à 49 de la SEQ ID NO : 1 et ladite séquence comprenant les séquences W-Y-F-[V/I/A] (SEQ ID NO : 71) et W-[I/V/A]-X4-G-S (SEQ ID NO : 69) où X4 est n'importe quel acide aminé, et une séquence de connexion entre la SEQ ID NO : 69 et la SEQ ID NO : 71 de 4 à 8 acides aminés, dans lequel les résidus [V/I/A] des SEQ ID NO : 69 et 71 ont une configuration D, et lequel peptide a une longueur d'environ 17 à environ 35 acides aminés.

2. Peptide selon la revendication 1, lequel peptide comprend la séquence W-Y-F-[V/I/A]-[K/R]-Xa-Xb-Xc-N-R-W-[V/I/A]-Xd-G-S-Xe-H (SEQ ID NO : 72) où Xa, Xb et Xc sont des acides aminés polaires, Xd est un acide aminé aliphatique et Xe est n'importe quel acide aminé.

3. Peptide selon la revendication 2, lequel peptide comprend la séquence W-Y-F-[V/I/A]-[K/R]-[S/N]-[S/Q/K]-[E/K/S/Q]-N-R-W-[V/I/A]-[L/V/A]-G-S-[A/G]-H (SEQ ID NO : 73).

4. Peptide selon l'une quelconque des revendications 1 à 3, lequel peptide comprend une séquence choisie dans le groupe constitué par les SEQ ID NO : 61 à 68.

5. Peptide selon la revendication 1, lequel peptide comprend une séquence de 24 résidus ayant une identité d'au moins 40 % avec la séquence aux positions 26 à 49 de la SEQ ID NO : 1, ladite séquence comprenant les séquences N/D-D-L-W-Y-F-[V/I/A] (SEQ ID NO : 21) et W-[I/V/A]-X4-G-S (SEQ ID NO : 69) où X4 est n'importe quel acide aminé, et une séquence de connexion entre la SEQ ID NO : 21 et la SEQ ID NO : 69 de 4 à 7 acides aminés, et lequel peptide a une longueur d'environ 25 à environ 35 acides aminés.

6. Peptide selon la revendication 5, lequel peptide comprend les séquences L-X1-X2-X3-N-D-L-W-Y-F-V/I (SEQ ID NO : 23) et W-I/V-X4-G-S (SEQ ID NO : 22) où X1, X2, X3 et X4 sont n'importe quels acides aminés.

7. Peptide selon la revendication 6, dans lequel X1 est l'alanine, l'acide aspartique ou l'acide glutamique, X2 est la lysine, l'asparagine ou l'acide aspartique, et X3 est la valine, l'isoleucine ou la glutamine.

8. Peptide selon la revendication 5, lequel peptide comprend les séquences L-A-K-V-N-D-L-W-Y-F-V (SEQ ID NO : 24) et W-I/V-X4-G-S (SEQ ID NO : 22) où X4 est un acide aminé hydrophobe, de préférence la leucine, la valine, l'alanine ou la méthionine.

9. Peptide selon l'une quelconque des revendications 5 à 8, lequel peptide comprend, consiste essentiellement en, ou consiste en, la séquence de la SEQ ID NO : 20, et dans lequel la valine à la position 19 est en configuration D et l'isoleucine à la position 27 est en configuration D.

10. Composition pharmaceutique comprenant un peptide selon l'une quelconque des revendications 1 à 9.

11. Peptide selon l'une quelconque des revendications 1 à 9, pour une utilisation en tant que médicament, de préférence en tant qu'antimicrobien, mieux encore en tant qu'antibactérien.

12. Méthode *in vitro* pour préparer un peptide selon l'une quelconque des revendications 1 à 9, laquelle méthode comprend une étape de mise en contact d'un peptide comprenant une séquence de 17 résidus ayant une identité d'au moins 40 % avec la séquence aux positions 33 à 49 de la SEQ ID NO : 1 et ladite séquence comprenant les séquences W-Y-F-[V/I/A] (SEQ ID NO : 71) et W-[I/V/A]-X4-G-S (SEQ ID NO : 69) où X4 est n'importe quel acide aminé, et une séquence de connexion entre la SEQ ID NO : 69 et la SEQ ID NO : 71 de 4 à 8 acides aminés, et dans laquelle le peptide a une longueur d'environ 17 à environ 35 acides aminés, avec une épimérase ayant une séquence d'acides aminés ayant une identité d'au moins 30 % avec la SEQ ID NO : 25 et dans laquelle 70 % des positions 14-15, 18, 20-24, 27, 58-60, 63, 83, 84, 87-90, 112, 115, 117, 120, 150, 152, 169, 176, 180, 181, 183, 204, 206-208, 214, 217, 220-225, 228, 230, 252, 254, 262, 289, 292, 296, et 309 de la SEQ ID NO : 25 sont conservés et toutes les positions 14-15, 18, 20-24, 27, 58-60, 63, 83, 84, 87-90, 112, 115, 117, 120, 204, 206-208, 214, 217, 220-225, 228, et 230 de la SEQ ID NO : 25 sont conservés.

13. Méthode *in vitro* selon la revendication 12, dans laquelle le peptide comprend une séquence de 24 résidus d'acides aminés ayant une identité d'au moins 40 % avec la séquence aux positions 26 à 49 de la SEQ ID NO : 1, ladite séquence comprenant les séquences N/D-D-L-W-Y-F-V/I (SEQ ID NO : 21) et W-I/V-X4-G-S (SEQ ID NO : 22) où X4 est n'importe quel acide aminé, et une séquence de connexion entre la SEQ ID NO : 21 et la SEQ ID NO : 22 de 4 à 7 acides aminés, et dans laquelle le peptide a une longueur d'environ 25 à environ 35 acides aminés.

14. Utilisation d'une épimérase de peptide SAM radicalaire ayant une séquence d'acides aminés ayant une identité d'au moins 30 % avec la SEQ ID NO : 25 et dans laquelle 70 % des positions 14-15, 18, 20-24, 27, 58-60, 63, 83, 84, 87-90, 112, 115, 117, 120, 150, 152, 169, 176, 180, 181, 183, 204, 206-208, 214, 217, 220-225, 228, 230, 252, 254, 262, 289, 292, 296, et 309 de la SEQ ID NO : 25 sont conservés et toutes les positions 14-15, 18, 20-24, 27, 58-60, 63, 83, 84, 87-90, 112, 115, 117, 120, 204, 206-208, 214, 217, 220-225, 228, et 230 de la SEQ ID NO : 25 sont conservés, pour la préparation d'un peptide selon l'une quelconque des revendications 1 à 9.

15. Cellule hôte recombinée comprenant un acide nucléique hétérologue codant un peptide comprenant une séquence de 17 résidus ayant une identité d'au moins 40 % avec la séquence aux positions 33 à 49 de la SEQ ID NO : 1 et comprenant les séquences W-Y-F-[V/I/A] (SEQ ID NO : 71) et W-[I/V/A]-X4-G-S (SEQ ID NO : 69) où X4 est n'importe quel acide aminé, et une séquence de connexion entre la SEQ ID NO : 69 et la SEQ ID NO : 71 de 4 à 8 acides aminés, et dans laquelle le peptide a une longueur d'environ 17 à environ 35 acides aminés, et un acide nucléique hétérologue codant une épimérase de peptide SAM radicalaire ayant une séquence d'acides aminés ayant une identité d'au moins 30 % avec la SEQ ID NO : 25 et dans laquelle 70 % des positions 14-15, 18, 20-24, 27, 58-60, 63, 83, 84, 87-90, 112, 115, 117, 120, 150, 152, 169, 176, 180, 181, 183, 204, 206-208, 214, 217, 220-225, 228, 230, 252, 254, 262, 289, 292, 296, et 309 de la SEQ ID NO : 25 sont conservés et toutes les positions 14-15, 18, 20-24, 27, 58-60, 63, 83, 84, 87-90, 112, 115, 117, 120, 204, 206-208, 214, 217, 220-225, 228, et 230 de la SEQ ID NO : 25 sont conservés, et capable de co-exprimer le peptide et l'épimérase.

16. Cellule hôte recombinée selon la revendication 15, dans laquelle le peptide comprend une séquence de 24 résidus ayant une identité d'au moins 40 % avec la séquence aux positions 26 à 49 de la SEQ ID NO : 1 et comprenant les séquences N/D-D-L-W-Y-F-[V/I/A] (SEQ ID NO : 21) et W-I/V-X4-G-S (SEQ ID NO : 22) où X4 est n'importe quel acide aminé, et dans laquelle le peptide a une longueur d'environ 25 à environ 35 acides aminés.

17. Méthode pour préparer un peptide selon l'une quelconque des revendications 1 à 9, laquelle méthode comprend la culture d'une cellule hôte recombinée selon la revendication 15 ou 16 dans des conditions convenant pour la co-expression du peptide et de l'épimérase, et la récupération du peptide ayant au moins deux acides aminés en configuration D.

18. Utilisation d'une cellule hôte recombinée selon l'une quelconque des revendications 15 et 16 pour préparer un peptide selon l'une quelconque des revendications 1 à 9.
